(19) Europäisches Patentamt
European Patent Office
Office européen des brevets

(11) EP 0 642 669 B1

(12) **EUROPEAN PATENT SPECIFICATION**

(45) Date of publication and mention of the grant of the patent:
**12.03.1997 Bulletin 1997/11**

(21) Application number: **93910302.4**

(22) Date of filing: **20.05.1993**

(51) Int Cl.[6]: **G01N 33/68**

(86) International application number:
**PCT/HU93/00030**

(87) International publication number:
**WO 93/24517 (09.12.1993 Gazette 1993/29)**

(54) **PEPTIDE SUB-LIBRARY KITS**

TESTSÄTZE VON PEPTID-UNTERBIBLIOTHEKEN

KITS DE SOUS-BANQUES DE PEPTIDES

(84) Designated Contracting States:
**AT BE CH DE DK ES FR GB GR IE IT LI LU MC NL PT SE**

(30) Priority: **21.05.1992 HU 168792**

(43) Date of publication of application:
**15.03.1995 Bulletin 1995/11**

(73) Proprietors:
- **FURKA, Arpad**
  **H-1074 Budapest (HU)**
- **SEBESTYEN, Ferenc**
  **H-1145 Budapest (HU)**

(72) Inventors:
- **FURKA, Arpad**
  **H-1074 Budapest (HU)**
- **SEBESTYEN, Ferenc**
  **H-1145 Budapest (HU)**

(74) Representative: **Deufel, Paul, Dr.**
**Müller-Boré & Partner**
**Patentanwälte**
**Grafinger Strasse 2**
**81671 München (DE)**

(56) References cited:
- **Science, Volume 249, issued 1990, July 27, American Association for the Advancement of Science; Washington (US); J.K. SCOTT and G.P. SMITH, "Searching for Peptide Ligands with an Epitope Library", pages 386-390, totality.**
- **Nature, Volume 354, No. 6348, issued 1991, November 7, London (GB), R.A. HOUGHTON et al., "Generation and Use of Synthetic Peptide Combinatorial Libraries for Basic Research and Drug Discovery", pages 84-86, totality.**
- **Nature, Volume 354, No. 6348, issued 1991, November 07, London (GB), K.S. LAM et al., "A New Type of Synthetic Peptide Library for Identifying Ligand-Binding Activity", pages 82-84, totality.**
- **International Journal of Peptide & Protein Research, Volume 37, issued 1991, Munksgaard, Copenhagen (DK), A. FURKA et al., "General Method for Rapid Synthesis of Multicomponent Peptide Mixtures", pages 487-493, totality.**
- **Science, Volume 251, issued 1991, February 1991, American Association for the Advancement of Science, Washington (US), S.P.A. FODOR et al., "Light-Directed, Spatially Addressable Parallel Chemical Synthesis", pp. 767-773/**
- **WO 93/22684**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The subject of the invention comprises special peptide kits, processes for their synthesis and methods for their application in identification of biologically active peptides (named "domino strategy") which can be utilized as potential drugs. Components of the kits are multicomponent mixtures of free or support bound peptides or peptide derivatives. Submission in logical order of components of the kits to binding experiments with living cells, tissues or proteins or execution of other kinds of biological or biochemical tests, and evaluation of their result, makes possible to determine the amino acid sequence of the bioactive peptides.

The invention is based on the highly efficient synthetic method introduced by the inventors and their colleagues: (i) Furka et al. Abstr. 14th Int. Congr. Biochem., Prague, Czechoslovakia, 1988, Vol 5, p. 47.; (ii) Furka et al. Abstr. 10th Int. Symp. Med.Chem., Budapest, Hungary, 1988, p. 288.; (iii) Furka et al. Int. J. Peptide Protein Res., 1991, 37, 487-493. This "portioning-mixing" method makes possible to prepare in a couple of days multicomponent peptide mixtures comprising, for example, all possible sequences of: tripeptides (8 thousand), tetrapeptides (160 thousand), pentapeptides (3.2 million) etc. It is a characteristic feature of this solid phase method that all peptide molecules formed on one bead of the solid support have the same sequence.

Later on Lam et al. (Nature, 1991, 354, 82-84) published a method identical with that of ours for the synthesis of support bound peptide mixtures and developed an identification procedure based on specific binding of protein molecules to the bead carrying the bioactive peptide, and determination of the sequence of the active peptide by using a sequenator. Houghten and his colleagues (Nature, 1991, 354, 85-86) described a slightly modified version of our synthetic method together with an iterative identification procedure applicable for mixtures of free peptides.

Fodor et al. (Science, 1991, 251, 767-773) also developed a new synthetic and identification method in which the peptides were synthesized in different but predetermined locations on the surface of a glass microscope slide. In the method of Scott and Smith (Science,1990, 249, 386-390) the peptide sequences are expressed on the surface of phage after inserting into its genome random nucleotide sequences.

WO 93/22684 concerns first order sub-library kits of oligomers (peptides and oligonucleotides) coupled to an unlabeled solid support.

Definitions and notations. The multicomponent peptide mixtures are usually called *peptide libraries.* Libraries synthesized by the "portioning-mixing" method can be deduced by varying amino acids in the different positions of peptides. This is experimentally accomplished by repeating the following operations: (i) dividing the support before coupling into equal portions, (ii) coupling a different amino acid to each portion, then (iii) mixing the portions. Before describing the invention in details some additional definitions are introduced.

The name *normal library* is used for mixtures formed by varying the same number of amino acids in all positions. If this is not the case, one can speak about *general libraries. Standard libraries* will represent a special case of the normal libraries where the 20 common amino acids are varied in all positions. Cysteine is often omitted and only the remaining 19 amino acids are varied. These mixtures are named *standard-C libraries.* A special combined library, containing a genetic series of standard libraries will be named *genetic library.* E.g., the genetic pentapeptide library includes all standard libraries from dipeptides to pentapeptides. The use of D-amino acids, derivatives of amino acids or non-common amino acids besides the common ones can be indicated by the name: *extended library.*

Any kind of library - except genetic libraries - may be considered as parent library and can be divided into sub-libraries. *Sub-libraries* are special partial libraries of parent libraries and can be synthesized by omitting the variation of the amino acids in one or more positions, while in the rest of positions they are varied like in the parent library. A non-varied position is occupied by the same amino acid in all peptides. Sub-libraries may be classified according their order: the first, second, third etc. order sub-libraries have one, two, three, etc. non-varied positions, respectively. Table 1 shows how the amino acids are varied in a first order standard pentapeptide sub-library.

Table 1.

| *c* | **Varied amino acids** |
|---|---|
| 1 | A,C,D,E,F,G,H,I,K,L,M,N,P,Q,R,S,T,V,W,Y |
| 2 | E |
| 3 | A,C,D,E,F,G,H,I,K,L,M,N,P,Q,R,S,T,V,W,Y |
| 4 | A,C,D,E,F,G,H,I,K,L,M,N,P,Q,R,S,T,V,W,Y |
| 5 | A,C,D,E,F,G,H,I,K,L,M,N,P,Q,R,S,T,V,W,Y |

Table 2 demonstrates variations in a second order general pentapeptide sub-library.

Table 2.

| *c* | **Varied amino acids** |
|---|---|
| 1 | A,C,D,E,F,G,M,N,P,Q,R,S,T,V,W,Y |
| 2 | A |
| 3 | L |
| 4 | A,L,M,N,P,Q,R,S,T,V,W,Y |
| 5 | A,C,D,E,F,G,H,I,K,L,M,N,P,Q,R,S,T,V,W,Y |

Examples for the synthesis of both the first and the second order sub-libraries of general libraries were described in the original presentation of the "portioning-mixing" method (cited above) although the terms introduced here were obviously not used.

The number of amino acid units in peptides is denoted by *n.* Coupling positions (*c*) are numbered from the C-terminus to express the order of couplings in the synthesis. The symbol *p* is used to indicate the number of amino acids varied in a coupling position. In the case of normal libraries *p* is constant while in general libraries may vary depending on coupling position. Symbol *k* is used to indicate the order of a sub-library.

Amino acids are denoted by one letter symbols: A (alanine), C (cysteine), D (aspartic acid), E (glutamic acid), F (phenylalanine), G (glycine), H (histidine), I (isoleucine), K (lysine), L (leucine), M (methionine), N (asparagine), P (proline), Q (glutamine), R (arginine), S (serine), T (threonine), V (valine), W (tryptophane), Y (tyrosine).

The symbol of sub-libraries comprises one or more pairs of a figure and a capital letter. The figure indicates the non-varied coupling position, while the letter is the symbol of the amino acid occupying it. The symbols of the first order sub-library of Table 1 and of the second order sub-library of Table 2 are 2E and 2A3L, respectively.

The number of peptides (*N*) in a standard peptide library depends on the number of residues: $N=20^n$, and that in one of its sub-library - in addition to *n* - depends on the order, too: $N=20^{(n-k)}$. Sub-libraries are classified according to the relative coupling positions of the non varied amino acids. For example, 2E and 2A3L are *c* 2 type first order and *c* 2,3 type second order sub-libraries, respectively. The number of types of sub-libraries (*T*) depends on both the order and the number of residues, and can be expressed by a simple formula.

$$T = \binom{n}{k}$$

For example, there are 10 types of second order pentapeptide sub-libraries which can be classified into two groups (Table 3):

Table 3.

| | | | | | |
|---|---|---|---|---|---|
| vicinal types: | *c* | 1,2 | 2,3 | 3,4 | 4,5 |
| disjunkt types: | *c* | 1,3 | 2,4 | 3,5 | |
| | *c* | 1,4 | 2,5 | | |
| | *c* | 1,5 | | | |

Collections of sub-libraries of the same order are called *sub-kits.* The *first order sub-library sub-kits,* alternatively named *first sub-kits* comprise all possible first order sub-libraries of a parent peptide library. The first order sub-library sub-kit (Table 4) belonging to a standard pentapeptide library has 100 components.

Table 4.

| |
|---|
| 1A,1C,1D,1E,1F,1G,1H,1I,1K,1L,1M,1N,1P,1Q,1R,1S,1T,1V,1W,1Y |
| 2A,2C,2D,2E,2F,2G,2H,2I,2K,2L,2M,2N,2P,2Q,2R,2S,2T,2V,2W,2Y |
| 3A,3C,3D,3E,3F,3G,3H,3I,3K,3L,3M,3N,3P,3Q,3R,3S,3T,3V,3W,3Y |
| 4A,4C,4D,4E,4F,4G,4H,4I,4K,4L,4M,4N,4P,4Q,4R,4S,4T,4V,4W,4Y |
| 5A,5C,5D,5E,5F,5G,5H,5I,5K,5L,5M,5N,5P,5Q,5R,5S,5T,5V,5W,5Y |

Another sub-kit can be derived by combining those first order sub-libraries which carry identical amino acids in their non-varied coupling position (and are found in a column of Table 4). This sub-kit is named *amino acid tester sub-kit.*

ΣA,ΣC,ΣD,ΣE,ΣF,ΣG,ΣH,ΣI,ΣK,ΣL,ΣM,ΣN,ΣP,ΣQ,ΣR,ΣS,ΣT,ΣV,ΣW,ΣY

A *second order sub-library sub-kit* comprises all possible second order sub-libraries of a given type or a collection of these. A 2,3 type second order sub-library sub-kit belonging to a standard pentapeptide library has 400 components (partially listed in Table 5. The vicinal type (see Table 3) second order sub-library sub-kit (belonging to the same parent library), which is particularly important in practice, has 1600 components. A collection of higher order sub-library sub-kits of a parent

Table 5.

| |
|---|
| 2A3A,2C3A,2D3A,2E3A,2F3A,2G3A,.....,2R3A,2S3A,2T3A,2V3A,2W3A,2Y3A |
| 2A3C,2C3C,2D3C,2E3C,2F3C,2G3C,.....,2R3C,2S3C,2T3C,2V3C,2W3C,2Y3C |
| 2A3D,2C3D,2D3D,2E3D,2F3D,2G3D,.....,2R3D,2S3D,2T3D,2V3D,2W3D,2Y3D |
| ........................................................... |
| ........................................................... |
| ........................................................... |
| 2A3V,2C3V,2D3V,2E3V,2F3V,2G3V,.....,2R3V,2S3V,2T3V,2V3V,2W3V,2Y3V |
| 2A3W,2C3W,2D3W,2E3W,2F3W,2G3W,.....,2R3W,2S3W,2T3W,2V3W,2W3W,2Y3W |
| 2A3Y,2C3Y,2D3Y,2E3Y,2F3Y,2G3Y,.....,2R3Y,2S3Y,2T3Y,2V3Y,2W3Y,2Y3Y |

peptide library is named *second sub-kit.* Collections of different order sublibrary sub-kits used in sequence determinations are called *sub-library kits.*

## Description of the invention

The object of the present invention is a kit of peptide mixtures for determination of the amino acid sequences of bioactive peptides in a parent peptide library comprising a "first sub-kit" which consists of a complete set of first order sub-libraries of said parent peptide library and a "second sub-kit" of peptide mixtures consisting of a complete set of vicinal and disjunct type second order and possibly higher order sub-libraries of the same parent peptide library or a partial library (occurrence library) of the same parent peptide library and possibly also comprising an amino acid tester sub-kit consisting of combined components of the "first sub-kit". In screening experiments done with a series of properly selected sub-libraries, sufficient information can be gathered to determine the sequences of the bioactive peptides.

A preferred embodiment of our invention is the kit wherein the components of the first sub-kit are special partial libraries of the parent peptide library in which the same amino acid occupies a certain position in all peptides while in the rest of positions the amino acids are varied like in the parent peptide library. If a screening experiment is carried out with a first order sub-library, and the response is negative, it means, that the amino acid occupying the non-varied coupling position does not occur in that position of the bioactive peptides. If the response is positive, on the other hand, the amino acid in question does occur in the non-varied position in at least some of the active peptides.

Components of the second sub-kit are special partial libraries of the parent peptide library in which two positions are occupied by the same two amino acids in all peptides while in the rest of positions the amino acids are varied like in the parent peptide library. Since the second order sub-libraries have two non varied positions they can be used to check the occurrence of a pair of amino acids in two coupling positions. Both the vicinal and disjunct type representatives. are important which can be used like dominoes in assembling the amino acid sequences of the bioactive peptides. Higher order sub-library sub-kits may also be included into the second sub-kit.

According to the invention components of the amino acid tester sub-kit are combined mixtures of all components of the first sub-kit in which the same amino acid occupies the non-varied position. The result of a screening experiment executed with a component of the amino acid tester sub-kit shows whether or not a certain amino acid occurs in any position of the bioactive peptides.

The number of amino acid residues of the peptides of parent peptide libraries might be preferably in the range of 2 to 25. Other ranges may be used as well.

According to the invention all kinds of amino acids can be used as subunits of peptides in the synthesis of the kit, like D-amino acids, unusual amino acids and/or derivatives of amino acids. The invention also refers to kits comprising mixtures of free or support-bound peptides.

The number of components in sub-library kits - and as a consequence the number of screening tests to be done - is in some cases quite considerable. Taking this in consideration we introduce in this invention labeling of sub-libraries. If some or all sub-libraries of a kit or sub-kit are labeled, the number of its components (and consequently the number of experiments in screening) can thoroughly be reduced, since the labeled sub-libraries can be mixed. The labels make the mixed sub-libraries distinguishable. For example, if 5 markers are used assigned to label coupling positions in the sub-kit demonstrated in Table 4, then sub-libraries of each column can be mixed resulting in 20 components instead of 100. Application of even a single marker in support bound kits (taking into consideration the unlabeled components) halves the number of components. The use of 20 markers assigned to amino acids makes possible to mix the components found in each now of Table 4 leading to only 5 components. By applying 20 markers the 400 components indicated in Table 5 can be replaced by 20 ones. The use of label carrying kits is advised when the kits are screened by binding experiments. Labeling can be applied in both support bound and free peptide kits. The applied colors have to be distinguished by eye or a suitable instrument (e.g. image processor). Among others the preferred colors may be: black, blue, green, cyan, red, brown, light blue, light green, light cyan, light red, yellow.

Thus, according to our invention, it is an advantageous form of kits of free peptides in which color and/or fluorescent marker groups are attached to the C- and/or N-termini of peptides.

In the case of support-bound kits, addition to color or fluorescence, the size of otherwise uniform size beads or the specific gravity of the beads can also be used as markers in the synthesis of labeled support bound kits. Glass and polymer beads, for example, can easily be distinguished as a consequence of the difference in their specific gravity.

We have found that kits are preferable whereby the components of the kits are mixtures of sub-libraries attached to solid supports differing in color and/or fluorescence and/or specific gravity and/or the size of the beads, where the color and/or the fluorescence and/or the specific gravity and/or the size of the beads refer to (i) the coupling position of the non-varied amino acid, (ii) the amino acid occupying the non-varied position (iii) both the non-varied amino acid and its position, thus making the mixed sub-libraries distinguishable.

A further embodiment of our invention are the processes for the synthesis of the above peptide sub-library kits. The processes were designed so as to use the minimum number of coupling steps in the synthesis and they comprise combination and/or repetition of at least one of the operations described below (Operations Op/i to Op/xv).

Operations Op/i to Op/xv.

(i) Portioning into *p* equal portions of a solid support or an amino acylated support or a mixed amino acylated support or a support carrying peptides or a support carrying a mixture of peptides where *p* is the number of amino acids varied in the parent peptide library in a given coupling position.

(ii) Removal of the terminal amino-protecting group then coupling a different protected amino acid to each of p portions.

(iii) Mixing *p* portions.

(iv) Removal of 1/(*n*-*r*+1) part of each of *p* portions giving samples B, and the residues which are named (if any remaining) samples A, where *r* and *n* are the number of amino acid residues already attached to the support and the number of amino acid residues in the peptides of the parent peptide library, respectively.

(v) Execution of operations (i) to (iii) on each of *p* samples.
(vi) Execution of operations (i)-(ii) on each of *p* portions.
(vii) Two sets of B-samples, each set comprising *p* samples, are combined in pairs containing the same amino acid residue in the non-varied position of the peptides to form *p* combined samples, named B-samples.
(viii) Coupling a different protected amino acid to each of *p* portions.
(ix) Execution of operations (i) to (iii) on one sample.
(x) Submission of each of *p* portions to removal of $R^2$ protecting group of the support and to coupling with a different color or fluorescent marker.
(xi) Removal of terminal amino-protecting group and coupling a different color or fluorescent marker to each of *p* portions.
(xii) Portioning of each of *p* equal samples into *p* equal portions.
(xiii) Removal of 1/(*n*-*r*) part of each of *p* portions otherwise the same as operation (iv).
(xiv) Submission of each of *p* portions to removal of $R^2$ protecting group of the support and to coupling with a different color or fluorescent marker.
(xv) Removal of 1/(*n*-*r*-1) part of each of *p* portions, otherwise the same as operation (iv).

According to a preferable embodiment of our invention there is provided a process for the synthesis of first sub-kit which comprises unlabeled free peptides in which the process is started with *p* equal samples of N-protected O-aminoacyl support, all samples differing in the aminoacyl residue.

(i) First, the samples are submitted to operation Op/iv, then samples A are consecutively submitted to operations Op/iii, Op/i and Op/ii and the resultant mixtures are used as starting samples in the next cycle of operations.
(ii) Samples B are submitted (*n*-*r*) times to operation Op/v then the samples are taken apart.
(iii) The cycle of operations (i) to (ii) of this procedure is repeated (*n*-2) time.

Finally all samples are submitted to deprotection and cleavage from the support.

A process for preparing a first sub-kit of peptides bound to unmarked support in which a sample of solid support, carrying blocked primary amino groups attached through a spacer, is first submitted to deprotection, operations Op/i and Op/viii, then the samples are treated according operations (i) to (iii) of the previous process and finally all samples are submitted to deprotection.

We also introduce a process for preparing a first sub-kit of free peptides carrying color or fluorescent labels at their C-terminus assigned to the amino acid residues occupying the non-varied positions.

(i) The process is started with *p* equal samples of polymer or glass supports comprising N-protected O-aminoacyl groups wherein the aminoacyl group is a color or fluorescent marker differing in color or fluorescence of the label of the support.
(ii) The samples are submitted to operation Op/xiii.
(iii) Samples A (if any remaining) are submitted to operation Op/v then are used as starting samples in the next cycle of operations.
(iv) Samples B are submitted to operation Op/ii, then mixed and submitted consecutively (*n*-*r*) times to operation Op/ix then the sample is taken apart.
(v). Operations (ii) to (iv) of this procedure are repeated (*n*-1) times.
(vi) All samples are submitted to deprotection and cleavage from the support.

According to our invention we describe a process for synthesizing a first sub-kit of a normal parent peptide library bound to color and/or fluorescent support where the color, or the fluorescence, or the size or the specific gravity of the beads or combinations of these characteristics are assigned to the amino acid residues occupying the non-varied position.

(i) The process is started with *p* equal samples of polymer and glass supports comprising free or protected primary amino groups wherein the beads are colored or fluorescent, differing in at least one of the mentioned characteristics.
(ii) The samples are submitted to operations (ii) to (v) of the previous process and finally all samples are submitted to deprotection.

In order to further enhance the economy of the synthesis of labeled sub library sub-kits, special solid supports were devised and introduced in this invention and methods are described for their use in the synthesis of labeled support-bound sub-kits and sub-kits of free peptides carrying the label at their C-terminus. Our experiences clearly show that much labour can be saved in the synthesis of sub-kits if the synthesis is started with unlabeled support and

the labels are introduced right before or right after building into peptides the non-varied amino acids. The solid supports (one for support bound kits and another one for kits of free peptides) devised for this purpose contain two kinds of protected primary amino groups. The two kinds of protecting groups can be removed independently. One of the protecting groups is removed to free the site of peptide synthesis the other one to allow coupling with the marker molecule. The color or fluorescent molecules applied as markers need to carry a functional group - among other possibilities a free carboxyl group - which make possible attachment to a free amino group. The advantages of this "in the course labeling" becomes clear if one compares to each other some characteristics of the three kinds of syntheses: (1) synthesis of unlabeled first sub-kits, (2) synthesis on labeled supports and (3) synthesis on unlabeled supports with "in the course introduction" of the label. When (1) is carried out, after the introduction of the non-varied amino acids the samples have to be treated individually. From this stage of the synthesis the number of coupling cycles to be executed are multiplied by a factor identical with the number of the varied amino acids (say 19). In (2) the synthesis is started with say 19 different solid supports which have to be treated individually (and executing 19 times the same operations) until the non-varied amino acids are introduced. After this stage the samples can be mixed and the number of operations in every position is reduced by a factor of 19. If (3) is used, one can start the synthesis with one sample. After introduction of the non-varied amino acids, the samples can be mixed again to follow with one sample, since the labels are already introduced (right before or right after executing coupling with the non-varied amino acids). So reduced number of operations are needed both before and after the introduction of the non-varied amino acids.

As a preferable embodiment in our invention of the type of synthesis described in the previous paragraph we provide an economical process for the synthesis of a first sub-kit of free peptides carrying color or fluorescent labels at their C-terminus assigned to the amino acid residues occupying the non-varied positions. The process is started with one sample of polymer or glass supports comprising O-acylated hydroxymethyl groups corresponding to the following general formula where $R^1$ and $R^2$ are protecting groups, $_j$, $_k$, and $_f$ are integers 1 to 6.

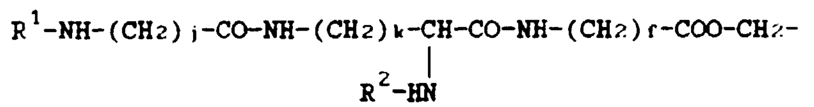

(i) The sample is submitted to operations Op/i and Op/ii.

(ii) The samples are then submitted to operation Op/iv.

(iii) Samples B are submitted to operation Op/x, then the samples are mixed and submitted (*n-r*) times to operation Op/ix and the sample is taken apart.

(iv) Samples A (if any remaining) are mixed then submitted to operations Op/i and Op/ii, then are used as starting samples in the next cycle of operations.

(v) The cycle of operations (ii) to (iv) of this procedure is repeated (*n*-1) times.

(vi) All samples are submitted to deprotection and cleavage from the support.

As a further preferable realization of our invention a very economical process is introduced for synthesizing a first sub-kit bound to color and/or fluorescent supports, using solid support comprising acylaminoalkyl groups corresponding to the following general formula 1 where $R^1$ and $R^2$ are protecting groups, $_j$, $_k$, and $_r$ are integers 1 to 6.

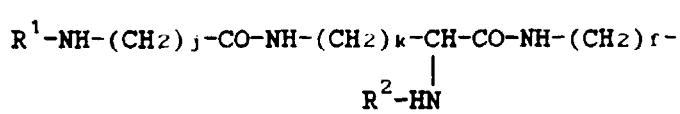

The color or fluorescent markers assigned to the amino acid residues occupying the non-varied position are attached to the support in the course of the peptide synthesis. The synthesis is started with one sample of support which is submitted to operations (i) to (v) of the previous procedure, finally, all samples are submitted to deprotection.

A process for synthesizing a first sub-kit of free peptides carrying color or fluorescent labels at their N-terminus assigned to the amino acid residues occupying the non-varied positions is a further preferable embodiment of our invention. N-terminal labeling is achieved by attaching color or fluorescent marker molecules (directly of *via* a spacer) to the N-terminal amino group of the peptides usually by acylation. The process is started with *p* equal samples of N-protected O-aminoacyl support, all samples differing in the aminoacyl residue.

(i) First, the samples are submitted to operation Op/iv.
(ii) Samples B are (*n*-*r*) times consecutively submitted to operation Op/v. Then operation Op/xi is executed and after mixing, the sample is taken apart.
(iii) samples A (if any remaining) are consecutively submitted to operations Op/iii, Op/i and Op/ii and the resultant mixtures are used as starting samples in the next cycle of operations.
(iv) the cycle of operations (i) to (iii) of this procedure is repeated (*n*-1) times then all samples are submitted to deprotection and cleavage from the support.

According to a preferable realization of our invention a process is introduced for synthesizing an amino acid tester sub-kit of free peptides comprising the following steps:

(i) The process is started with *p* equal samples of N-protected O-aminoacyl support, all samples differing in the acylamino residue.
(ii) First, the samples are submitted to operation Op/iv. On samples A (if any remaining) operations Op/iii, Op/i and Op/ii are consecutively executed then the samples are used in the next cycle operations. Samples B together with the resultant B samples of of the previous cycle (if executed) are submitted to operation Op/vii.
(iii) If $r < n$ samples B are submitted to operation Op/v otherwise are taken apart.
(iv) Cycle comprising operations (ii) to (iii) of this procedure are repeated (*n*-1) times.
(v) Finally, the resultant B samples are submitted to deprotection and cleavage from the support.

Another preferable process in our invention is the synthesis of a sub-kit comprising all vicinal type second order sub-libraries of a parent peptide library of unlabeled soluble peptides, in which the process is started with *p* equal samples of O-acylamino support, all samples differing in the acylamino residue.

(i) The samples are submitted to operation Op/xi. Samples A (if any remaining) are consecutively submitted to operations Op/iii, Op/i, Op/ii then the resultant A samples are used in the next cycle of operations. Samples B are submitted to operation Op/vi then the unmixed resultant samples are one by one submitted to *n*-*r* consecutive cycles of operations Op/i to Op/iii. All resultant samples originating from samples B are taken apart.
(ii) Cycle of operations (i) of this claim is repeated *n*-2 times.
(iii) All samples are submitted to cleavage and deprotection. Our invention includes a process for synthesizing a sub-kit bound to unlabeled support comprising all vicinal type second order sub-libraries of a parent peptide library, in which a sample of solid support, carrying blocked primary amino groups attached through a spacer, is consecutively submitted to deprotection, operations Op/i and Op/viii, then the samples are treated according operations (i) to (ii) of the previous procedure, and finally, all samples are submitted to deprotection.

According to a preferable embodiment of our invention, a process is provided for synthesizing a sub-kit comprising all vicinal type second order sub-libraries of a parent peptide library of soluble peptides, carrying color or fluorescent labels at their C-terminus, assigned to the amino acid residue occupying the lower coupling position of the two vicinal non-varied positions, in which the process is started with p equal samples of solid support comprising N-protected O-aminoacyl groups wherein the aminoacyl group is a color or fluorescent marker, differing in color or fluorescence of the label of the support.

(i) first, operation Op/xv is executed. Samples A (if any remaining) are submitted to operation Op/v then the resultant A samples are used in the next cycle of operations. Samples B are consecutively submitted to operations Op/ii, Op/iii), Op/i) and Op/ii), then the resultant samples are submitted to *n*-*r* consecutive cycles of operation Op/v) then the samples are taken apart.
(ii) cycle of operations (i) of this procedure is repeated (*n*-2) times.
(iii) Finally all samples are submitted to cleavage and deprotection.

A process for synthesizing a sub-kit comprising all vicinal type second order sub-libraries of a normal parent library of peptides bound to color or fluorescent supports, when the color or fluorescence of the support is assigned to the amino acid residue occupying the lower coupling position of the two vicinal non-varied positions is a further preferable embodiment of our invention. The process is started with *p* equal samples of polymer and glass supports comprising free or protected primary amino groups, wherein the beads are colored or fluorescent, differing in color or fluorescence of the label of the support. The samples are submitted to operations (i) to (ii) of the previous procedure, finally all samples are submitted to deprotection.

As an economical embodiment of our invention we introduce a process for synthesizing a sub-kit comprising all vicinal type second order sub-libraries of a normal parent library of peptides bound to color or fluorescent supports,

when the color or fluorescence of the support is assigned to the amino acid residue occupying the lower coupling position of the two vicinal non-varied positions, and in which the process is started with a single deprotected sample of polymer and glass support comprising acylaminoalkyl groups corresponding to the following general formula 1 where $R^1$ and $R^2$ are protecting groups, $_j$, $_k$, and $_f$ are integers 1 to 6.

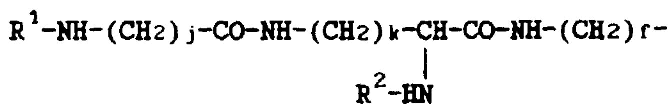

The color or fluorescent labels are attached to the support in the course of the peptide synthesis.

(i) The sample is submitted to operations Op/i and Op/ii.
(ii) The samples are submitted to operation Op/xiii. Samples A (if any *remaining)* are consecutively submitted to operations Op/iii, Op/i, Op/ii then the resultant A samples are used in the next cycle of operations. Samples B are submitted to operation Op/x, then mixed and submitted to operations Op/i and Op/ii then the resultant samples are submitted to *n-r* consecutive cycles of operation Op/v then the samples are taken apart.
(iii) Cycle of operations (ii) of this procedure is repeated *n*-2 times.
(iv) Finally all samples are submitted to deprotection.

According to our invention we describe an economical process for the synthesis of a sub-kit comprising all vicinal type second order sub-libraries of a normal parent library of peptides bound to color or fluorescent supports, when the color or fluorescence of the support is assigned to the amino acid residue occupying the lower coupling position of the two vicinal non-varied positions. The process is started with a single deprotected sample of solid support comprising O-acylated hydroxymethyl groups corresponding to the following general formula where $R^1$ and $R^2$ are protecting groups, $_j$, $_k$, and $_f$ are integers 1 to 6

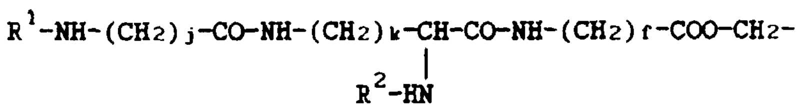

and the color or fluorescent labels are introduced in the course of the peptide synthesis. The sample is submitted to operations (i) to (iii) of the previous procedure then all samples are submitted to deprotection and cleavage.

According to our invention, determination of the amino acid sequences of the bioactive peptides of a parent peptide library can be accomplished in three stages by using the domino strategy. There are no special requirements concerning the screening experiments used.

(i) First a screening experiment is carried out with all components of the amino acid tester kit. The positive answers show which amino acids occur in the active peptides regardless of their coupling position. The list of these amino acids is named *amino acid occurrence list.*
(ii) In the second stage, the screening experiments are done with a properly selected set of components of the first sub-kit to determine the coupling positions of the amino acids of the amino acid occurrence list. If, for example, alanine is present in the amino acid occurrence list of a pentapeptide parent library, one has to do screening experiments - among others - with the following first order sub-libraries: 1A, 2A, 3A, 4A and 5A. The result of these experiments show which amino acids vary in the different coupling positions of the bioactive peptides. These amino acids together with their coupling positions define a partial library of the parent one, named *occurrence library.* All bioactive peptides are present in the occurrence library, together with inactive ones but the total number of components is highly reduced relative to that of the parent library.
(iii) The third stage comprises determination of the sequences by use of the selected components of the second sub-kit. To exemplify this, let's suppose that the amino acid variations in the coupling positions 1 and 2 of the occurrence library are A, G and P, T, respectively. One has to determine whether P or T or both follow A, and, whether P or T or both follow G in different active sequences. Screening tests with 1A2P, 1A2T, 1G2P and 1G2T answer the questions. Then the amino acids varied in coupling positions 2,3 and 3,4 etc. of the occurrence library have to be similarly examined. Two different approaches can be followed in these experiments: (a) using selected components of the presynthesized second sub-kit of the parent peptide library or (b) synthesizing and using the

second sub-kit the occurrence library.

If labeled support-bound sub-library kits are screened one has to observe the color or/and size etc. of the beads which bind macromolecules or cells. When labelled soluble kits are screened the gained color or/and fluorescence of the examined macromolecules, cells or tissues guides the experimenter in determining which component of the kit binds to the target.

The applicants also provide polymer and glass supports comprising free or protected primary amino groups, wherein the beads are colored or fluorescent. Color or fluorescent supports can be prepared by coupling a color or fluorescent marker molecule (and a spacer) to the surface of the beads. Those molecules can be used for this purpose which carry a functional group for attachment to the support - preferably a carboxyl group - and have another functional group - preferably an amino group - for coupling to the peptides or to a spacer. These color or fluorescent amino acids form a bridge between the solid support and the peptides.

Polymer and glass supports, comprising acylaminoalkyl groups corresponding to the following general formula where $R^1$ and $R^2$ are protecting groups, $_j$, $_k$, and $_f$ are integers 1 to 6,

$$R^1\text{-NH-}(CH_2)_j\text{-CO-NH-}(CH_2)_k\text{-}\underset{\underset{R^2\text{-HN}}{|}}{CH}\text{-CO-NH-}(CH_2)_f\text{-}$$

allow the introduction of the color or fluorescent label in the course of the synthesis of bound and labeled sub-library sub-kits. The built in marker molecules will form a bridge between the beads and the peptides to be synthesized.

Solid supports, carrying N-protected O-aminoacyl groups wherein the aminoacyl group is a color or fluorescent marker, are use in synthesis of sub-library kits comprising peptides labeled at their C-terminal part with color or fluorescent markers. The markers are color or fluorescent amino acid type molecules which form a bridge between the surface of the beads and the synthesized peptides and they remain attached to the peptides when the peptides are removed from the support at the end of the synthesis.

We also introduce solid supports comprising O-acylated hydroxymethyl groups corresponding to the following general formula where $R^1$ and $R^2$ are protecting groups, $_j$, $_k$, and $_f$ are integers 1 to 6.

$$R^1\text{-NH-}(CH_2)_j\text{-CO-NH-}(CH_2)_k\text{-}\underset{\underset{R^2\text{-HN}}{|}}{CH}\text{-CO-NH-}(CH_2)_f\text{-COO-}CH_2\text{-}$$

We also introduce a special azo dye 4-[4-[N-Ethyl-N-[3-[*tert*-butyloxy-carbonyl]aminopropyl]amino]phenylazo]benzoic acid (EPAP) and O-acylated as well as N-acylated polymer or glass supports derived by its use.

Advantages of the invention. The sub-library kits introduced in this invention may be regarded as new, generally applicable tools in screening of peptide mixtures. The "domino strategy" based on these kits is very economical and involves no condition of any kind concerning the method used in the screening experiments. Furthermore, support bound or free sub-library kits are promising equally good results, and the constituent amino acids are by no means restricted to the common natural ones. On the contrary, our synthetic and screening strategy can be applied to mixtures of any other kinds of bio-oligomers that can be synthesized by the solid phase method.

Support bound mixtures can be screened without the use of expensive sequenators and the method is independent of the current state of art of sequence determination of peptides incorporating unusual amino acids. Moreover, since no degradation is needed in sequence determinations the components of the kits are easily regenerated opening the possibility for a very economical multiple usage.

In screening of free peptide mixtures the use of sub-library kits and the "domino strategy" makes possible to determine the sequence of bioactive peptides in less screening steps than other methods do. Moreover, since no peptide synthesis is needed in determinations the method can easily be used by biologists lacking the special skills of peptide chemists.

The described syntheses of unlabeled sub-library kits are optimized so to use the less possible number of coupling cycles. Further reduction in the number of coupling cycles is achieved by using labeled polymers in the synthesis. The economy of the synthesis is even more increased by using special supports designed to allow introduction of the labels in the course of peptide synthesis.

The following examples are intended to demonstrate - but by no means limit - our invention.

Examples

Example 1 N-Ethyl-N-[3-[*tert*-butyloxycarbonyl]aminopropyl]aniline

N-Ethyl-N-[3-aminopropyl]aniline (S.L.Shapiro et al., J.Amer.Chem. Soc. 1959, 81, 3083-3088) (1.78 g; 10 mmol) is reacted with di-tert-butyl dicarbonate according to L.Moroder et al. (Hoppe Seyler's Z.Physiol.Chem. 1976, 357, 1651-1653) to yield an oily product (90%).

Example 2

4-[4-[N-Ethyl-N-[3-[*tert*-butyloxycarbonyl]aminoro]amino]phenylazo]benzoic acid (EPAP)

4-Aminobenzoic acid (1.23 g; 9 mmol) dissolved in a mixture of ethanol (25 mL) and cc. hydrochloric acid (5 mL) is reacted with sodium nitrite (0.62 g; 9 mmol), the temperature being kept between 0° and 5°C. To the ice-cold solution of the diazonium salt, sodium acetate (4.1 g) then N-ethyl-N-[3-[*tert*-butyloxycarbonyl]-aminopropyl]aniline (Example 1) (2.5 g; 9 mmol) in ethanol (6 mL) are added. The mixture is stirred for 4 h at room temperature then allowed to stand overnight. The brick-red precipitate is filtered, washed with water and dried. The crude product is recrystallized from toluene to yield 2.4 g (62%).

Example 3

Assortment of polymer resin beads by size

Aminomethylated styrene-divinyl benzene (99:1) copolymer beads (10 g) (Mitchell et al., J.Amer.Chem.Soc. 1976, 98, 7357-7362; 1 meq N/g; 200-400 mesh [0.038-0.075 mm]) are sieved by using an upper and a lower sieve, 230 mesh (0.063 mm) and 325 mesh (0.045 mm), resp. The sieving results in three fractions of beads: 1. fine (325-400 mesh, 0.038-0.045 mm; 2 g), 2. medium (230-325 mesh, 0.045-0.063 mm; 5 g), 3. coarse (200-230 mesh, 0.063-0.075 mm; 3 g).

Example 4

Coarse Boc-Gaba-aminomethyl resin beads

Coarse aminomethyl resin beads (Example 3; 1 g) are swollen in dichloromethane (DCM), filtered, and a mixture of ethyl diisopropyl amine (EDIA) and DCM (1:9, v/v) (10 mL) is added. The mixture is shaken for 5 min, filtered, and the treatment with the former reagent solution is repeated. The mixture is allowed to stand for 15 min then filtered. To a solution of 0.81 g (4 mmol) Boc-$\gamma$-aminobutyric acid (Boc-Gaba) in DCM (5 mL), 1-hydroxybenzo - triazole (HOBt) (0.8 g; 5.2 mmol) then diisopropyl carbodiimide (DIC) (0.62 mL; 4 mmol) is added. The aminomethyl resin is suspended in this solution. The mixture is shaken for 3 h at room temperature, then the resin is filtered and washed (4x DCM, 2x ethanol [EtOH]). The beads which give negative test with ninhydrin (Kaiser et al., Anal.Biochem. 1970, 34, 595-598) are dried over $P_2O_5$.

Example 5

Fine Boc-Gaba-EPAP-aminomethyl resin beads

Fine aminomethyl resin beads (Example 3; 1 g) are swollen in DCM, filtered, and a mixture of EDIA and DCM (1:9, v/v) (10 mL) is added. The mixture is shaken for 5 min, filtered, and the washing with the former reagent solution is repeated. The mixture is allowed to stand for 15 min then filtered. To a solution of 1.70 g (4 mmol) EPAP (Example 2) in dimethyl formamide (DMF (30 mL), HOBt (0.8 g; 5.2 mmol) then DIC (0.62 mL; 4 mmol) is added. The aminomethyl resin is suspended in this solution. The mixture is shaken for 3 h at room temperature. The red resin beads are filtered and washed (3x DMF, 3x DCM), filtered and a mixture of DCM and trifluoroacetic acid (TFA) (2:1, v/v) (10 mL) is added. The mixture is shaken for 5 min, filtered, again DCM/TFA mixture (10 mL) added and, after shaking for 30 min, the mixture is filtered, washed with DCM, MeOH, DCM, MeoH, 2x DCM. The resin is treated with EDIA and, after washing, with Boc-Gaba, HOBt and DIC according to Example 4.

Example 6

Nε-[Boc-Gaba]-Nα-Fmoc-L-Lysyl aminomethyl resin beads (BGFL-aminomethyl resin beads)

Coarse aminomethyl resin beads (Example 3; 1 g) are treated as described in Example 4. The difference is that instead of Boc-Gaba, Na-Fmoc-Nε-Boc-L-lysine (1.87 g; 4 mmol) is used. After coupling, the resin is reacted with DCM/TFA mixture (Example 5) then with EDIA and, after washing, with Boc-Gaba, HOBt and DIC according to Example 4.

Example 7

EPAP-Hydroxymethyl resin

Chloromethylated styrene-divinyl benzene (99:1) copolymer beads (Bio-Rad; 200-400 mesh; 1.26 meq Cl/g) (1 g) are swollen in DMF (30 mL) then EPAP (Example 2) (1.53 g; 3.6 mmol) and potassium fluoride (KF) (0.3 g) are added. The mixture is stirred for 24 h at 50° then washed with DMF, DCM and EtOH. The red beads are dried over $P_2O_5$.

Example 8

Nε-Boc-Nα-Fmoc-L-Lysyl-[4-aminobutyryl]-hydroxymethyl resin (BFLG-hydroxymethyl resin)

Chloromethylated styrene-divinyl benzene (99:1) copolymer beads (see Example 7) (1 g) are swollen in DMF (30 mL) then Boc-Gaba (0.73 g; 3.6 mmol) and KF (0.3 g) are added. The mixture is stirred for 24 h at 50°, washed with DMF and DCM, filtered, and a mixture of DCM and TFA (2:1, v/v) (10 mL) is added. The mixture is shaken for 5 min, filtered, shaken with a new portion (10 mL) of DCM/TFA, filtered and washed with DCM, MeOH, DCM, MeOH and 2x DCM. The resin is treated with EDIA and, after washing, with Na-Fmoc-Nε-Boc-L-lysine, HOBt and DIC as described in Example 4 (lysine derivative is used instead of Boc-Gaba).

Example 9

Mixing of resin beads

Portions of resin beads (including starting resins, aminoacyl- and peptidyl-resins) are swollen in a mixture of DCM and DMF (2:1, v/v), poured and rinsed into a common polyethylene (PE) vessel. Swelling and rinsing require 5 mL liquid/100 mg of resin sample. The resultant mixture is shaken for 15 min then is ready for immediate portioning.

Example 10

Portioning of resin beads

A resin sample swollen in DCM/DMF (Example 9) is portioned by volume, using a 5 mL PE measuring pipette, into the reaction vessels (PE tubes fitted with plastic frit).

Example 11

Coupling of a Boc-amino acid to the resin beads

A sample of one of polymer supports described in Examples 4-8 or an Na-Boc-aminoacyl- or an Na-Boc-peptidyl resin containing 0.1 mmol of protected α-amino groups is placed into the reaction vessel (see Example 10). During the forthcoming operations the resin is shaken with different reagent solutions or washing solvents and at the end of operations the liquids are removed by suction. The operations are: A/ Swelling (in the case of dry resin sample): 2x3 mL DCM, 2x5 min. B/ Removal of Boc-groups: DCM/TFA 2:1 (v/v) 3 mL, 2 min; the same mixture, 3 mL, 30 min. C/ Washing: DCM, MeOH, DCM, MeOH, DCM, DCM, 3 mL and 5 min each. D/ Deprotonation: DCM/EDIA 9:1 (v/v) 3 mL, 2 min; the same mixture, 3 mL, 8 min. E/ Washing: DCM 3x3 mL, 9 min each. F/ Preparation of active ester: Boc-amino acid (side chain protecting groups used: OBzl for Asp and Glu, Bzl for Ser, Thr and Tyr, Z for Lys, Tos for Arg and His) (0.4 mmol) is dissolved in DCM/DMF 3:1 (v/v) (2 mL) in a separate vessel (in the case of Boc-Gln, Boc-Asn, Boc(Tos)-Arg and Boc(Tos)-His, the solvent is 2 mL of DMF) and, with stirring, HOBt (0.08 g; 0.52 mmol) then DIC (0.062 mL; 0.4 mmol) are added. G/ Coupling: The solution of active ester (see F) is added to the resin (prepared in section E of this Example), the mixture is shaken for 3 h at room temperature. H/ Washing: DMF (3x) then DCM (3x), 3 mL and 5

min each.

Example 12

Removal of Fmoc-groups

Resins carrying 0.1 mmol of a peptide mixture prepared by the use of resins described in Examples 6 or 8 as starting materials, are swollen in DCM then DMF (2x3 mL, 3 min each), shaken with DMF/piperidine (1:1, v/v) (3 mL) for 20 min, filtered and washed with 2x3 mL of DMF, dioxane/water 1:1, DMF and DCM, 5 min each.

Example 13

Labeling of peptides bound to BGFL-aminomethyl resin beads

Resin sample carrying a peptide mixture prepared by the use of BGFL-aminomethyl resin (Example 6) as starting materials, with free α-amino groups on the linker lysine residue after removal of Fmoc-groups as described in Example 12, is subjected to operations described in Example 11 F-H. Instead of a Boc-amino acid, however, marker molecules like 5-dimethylamino-1-naphthalenesulfonyl-Gaba (Dns-Gaba), 7-hydroxycoumarin 4-acetic acid (HCAA) and EPAP (see Example 2) are used.

Example 14

Labeling of peptides to be cleaved from BFLG-hydroxymethyl resin beads

Resin sample carrying a peptide mixture prepared by the use of BFLG-hydroxymethyl resin (Example 8) as starting material, with free α-amino groups on the linker lysine residue after removal of Fmoc-groups described in Example 12, is treated according to Example 13.

Example 15

Labeling of peptides at their N-termini

Resin sample carrying a peptide mixture prepared by the use of Boc-aminoacyl resin esters as starting materials, is subjected to operations described in Example 11 B-E, then to operations of Example 11 F-H (coupling with one of marker molecules; see Example 13).

Example 16

Removal of protecting groups from resin-bound peptides

Resin-bound peptide mixtures prepared by the use of resin beads described in Examples 4, 5 and 6 as starting materials, are treated with a mixture of trifluoromethanesulfonic acid (TFMSA) and TFA according to Yajima et al. (J. Chem.Soc., Chem.Commun. 1974, 107-108), then washed with ether and MeOH, dried over $P_2O_5$.

Example 17

Cleavage of peptides from the resin

Resin (50 mg), carrying peptide mixtures prepared from resins described in Examples 7 or 8, or from commercially available Boc-aminoacyl resin esters, is treated with TFMSA/TFA (see Example 16), the resin is filtered off, then the acidic reaction mixture is added to dry ether (25 mL). The mixture is allowed to stand overnight at -20°C. The precipitate is collected by filtration, washed twice with ether and dried over KOH.

Example 18

Mixing, portioning, coupling

Samples of 19 different protected aminoacyl resins are mixed (Example 9) then portioned (Example 10) resulting

in 19 new samples which are separately coupled with one of the following amino acid derivatives according to Example 11: Boc-Ala, Boc(Tos)-Arg, Boc-Asn, Boc-Asp(OBzl), Boc-Gln, Boc-Glu(OBzl), Boc-Gly, Boc(Tos)-His, Boc-Ile, Boc-Leu, Boc(Z)-Lys, Boc-Met, Boc-Phe, Boc-Pro, Boc(Bzl)-Ser, Boc(Bzl)-Thr, Boc-Trp, Boc(Bzl)-Tyr, Boc-Val.

Example 19

Synthesis of the first sub-kit of a free standard-C tetrapeptide library

/i/ 2.0 mmol samples of the 19 kinds of Boc-amino acid resin esters are submitted to 3 consecutive cycles of operations described as A-cycle in the next paragraph of this Example.
/ii/ A-cycle: 0.5 mmol of each sample is removed and the 19 removed samples are taken apart for use in B-cycles. The residual parts of samples are subjected to operations described in Example 18 to produce 19 new samples.
/iii/ Each of the 3x19 samples removed in A-cycles is repeatedly submitted to the following B-cycles of operations until it attains the tetrapeptide size.
/iv/ B-cycle: Each sample is divided into 19 equal portions, one of the 19 common Boc-amino acids is coupled to each portion then the portions are mixed resulting in 19 samples.
/v/ The 19 final samples of /ii/ and the 3x19 final samples of /iii/ are cleaved from the support according to Example 17.

Example 20

Synthesis of the first sub-kit of a resin-bound standard-C tetrapeptide library

19 kinds of Boc-aminoacyl-Gaba-aminomethyl resin beads are prepared by the use of different Boc-amino acids and the resin described in Example 4, according to Example 11. 2.0 mmol samples of the resultant 19 resins are submitted to 3 consecutive A-cycles (Example 19/ii) then operations described in Example 19 iii and iv. The protecting groups from the 76 resin-bound final samples are removed according to Example 16.

Example 21

First sub-kit of a free standard-C tetrapeptide library carrying color-and/or fluorescence-labels at the C-termini of peptides starting from labeled resin esters

/i/ 19 kinds of color- and/or fluorescence-labeled resin esters (2.0 mmol each) are used as starting materials and submitted to 4 consecutive main cycles.
/ii/ Main cycle: 0.5 mmol of each resin sample is removed and taken apart as B-sample. Each of the remaining samples (A-samples; if any remaining) is submitted to the following operations: dividing into 19 equal parts, each part is coupled with a different Boc-amino acid according to Example 11 then the samples are mixed and taken apart for using as starting material in the next cycle. Each of B-samples is coupled with a different Boc-amino acid according to Example 11, then the samples are mixed. If the peptides are shorter then tetrapeptides, the sample is submitted to consecutive inner cycles until the tetrapeptide size is attained.
/iii/ Inner cycle of the main cycle: The sample is divided into 19 parts, each part is coupled with a different Boc-amino acid then the samples are mixed.
/iv/ Finally the peptides of the 4 samples are cleaved from the support according to Example 17.

Example 22

First sub-kit of a resin-bound standard-C tetrapeptide library synthesized on beads differing from each other by their color, fluorescence and/or size

19 kinds of colorless (Example 4), colored (Example 5) and fluorescent derivatives of fine and coarse aminomethyl resin beads (Example 3) are used as staring materials and submitted to operations described in paragraphs /ii/ and /iii/ of Example 21. The protecting groups from the 4 final samples are removed according to Example 16.

Example 23

First sub-kit of a free standard-C tetrapeptide library carrying color or fluorescent labels at the C-termini of peptides (labeling in the course)

BFLG-hydroxymethyl resin (Example 8) is divided into 19 equal portions and each of them is submitted to operations described in Example 11 resulting in 19 different Boc-aminoacyl-BFLG-hydroxymethyl resins, 2.0 mmol of each.

/i/ The resultant resins are submitted to 3 consecutive E-cycles of the following operations.
/ii/ E-cycle: 0.5 mmol of each sample is removed. The 19 removed samples are taken apart for use in F-cycles. The residual parts of the samples are mixed together, divided into 19 equal parts, and each new part is coupled with a different Boc-amino acid.
/iii/ Fmoc-group is removed from each sample obtained after the last E-cycle according to Example 12 then each sample is coupled with a different color and/or fluorescence marker (see Example 14) and the samples are mixed together to produce one of the 4 final samples.
/iv/ Each of the sets of 19 samples removed and taken apart in the different E-cycles, is treated as described in /iii/ of this Example, then repeatedly submitted to the F-cycles of operations, described in the next paragraph, until it attains the tetrapeptide size and produce 3 final samples.
/v/ F-cycle: The sample is divided into 19 equal portions, each portion is coupled with a different Boc-amino acid then the new portions are mixed together.
/vi/ The peptides of the 4 final samples (originated in /iii/ and /iv/) are cleaved from the support according to Example 17.

Example 24

Labeled first sub-kit of a resin-bound standard-C tetrapeptide libra y (labeling in the course)

19 kinds of Boc-aminoacyl resin beads (2.0 mmol of each) are prepared according to Example 11, using BGFL-aminomethyl resin beads (Example 6). On these Boc-aminoacyl resins, operations described in Example 23 i to 23 v are executed. The protecting groups from the 4 final samples are removed according to Example 16.

Example 25

First sub-kit of a free normal pentapeptide library carrying color labels at the N-termini of peptides

/i/ 2.0 mmol samples of 10 different Boc-amino acid resin esters (resin esters of Boc-Ala, Boc(Tos)-Arg, Boc-Asp-OBzl, Boc-Glu(OBzl), Boc-Gly, Boc(Z)-Lys, Boc-Phe, Boc-Pro, Boc(Bzl)-Ser and Boc(Bzl)-Tyr) are submitted to 4 consecutive G-cycles of the following operations.
/ii/ G-cycle: 0.4 mmol of each sample is removed and taken apart for use in H-cycles. The residual parts of samples are mixed together, divided into 10 portions, then each portion is coupled with a different Boc-amino acid (Example 11). The resultant samples (except the remainder produced in the last cycle) are used as starting samples in the next cycle.
/iii/ Each of the sets of 10 samples removed in G-cycles is repeatedly submitted to the following H-cycles of operations until it attains the pentapeptide size.
/iv/ H-cycle: Each sample is submitted to the following operations: dividing into 10 equal portions, one of the Boc-amino acids listed in /i/ of this Example is coupled to each portion, then the sets of 10 resultant portions are mixed together.
/v/ The 10 remainder samples of the 4th G-cycle as well as the 4x10 samples produced in H-cycles are one by one coupled with Boc-Gaba (likewise Example 11) resulting in 5 sets of 10 samples. Then each set is treated as follows: each of the 10 samples is coupled with one of different color markers as described in Example 15, then mixed together to produce 1 final sample.
/vi/ The final samples obtained in /v/ are cleaved from the support according to Example 17.

Example 26

Amino acid tester sub-kit of a free standard-C tripeptide library

/i/ 19 samples (numbered from 1 to 19) of 2.1 mmol Boc-amino acid resin esters - containing the amino acid

residues in order of A, D, E, F, G, H, I, K, L, M, N, P, Q, R, S, T, V, W, Y - are submitted to 3 consecutive I-cycles of operations.

/ii/ I-Cycle: 0.7 mmol of each sample is removed (inheriting the number of the original sample) and the samples are taken apart for use as starting samples in operations described in paragraph /iii/. The residual parts of samples (if any remaining) are mixed together, divided into 19 numbered portions, then each portion is coupled with a different Boc-amino acid in order described in paragraph /i/. The resultant numbered samples are used as starting samples in the next I-cycle.

/iii/ Each of 19 samples removed in the first I-cycle is treated as follows: the sample is divided into 19 portions. Each portion is coupled with a different Boc-amino acid then mixed. In this way 19 new numbered samples are formed.

/iv/ Each of the 19 samples removed in the second I-cycle is united with its identically numbered pair obtained in paragraph /iii/ and the new sample is carrying the same number. Each of 19 united samples is treated as follows: the sample is divided into 19 new portions, and each portion is coupled with a different Boc-amino acid then mixed to one sample carrying the original number.

/v/ Each of the 19 samples obtained in /iv/ is united with the identically numbered sample removed in the third I-cycle to produce 19 final samples.

/vi/ The peptides of 19 final samples are cleaved from the support according to Example 17.

Example 27

Amino acid tester sub-kit of a resin-bound standard-C tripeptide library

19 samples (numbered from 1 to 19) of 2.1 mmol Boc-aminoacyl-Gaba-aminomethyl resin (see Examples 4 and 11) - containing the amino acid residues in order of A, D, E, F, G, H, I, K, L, M, N, P, Q, R, S, T, V, W, Y - are submitted to 3 consecutive I-cycles of Example 26 then operations described in Example 26 iii to 26 v are executed. The protecting groups from the 19 resin-bound final samples are removed according to Example 16.

Example 28

Second order vicinal sub-library sub-kit of a free standard-C tetrapeptide library

1.5 mmol samples of the 19 kinds of Boc-amino acid resin esters are submitted to 3 consecutive main cycles of the following operations.

Main cycle: 0.5 mmol of each sample is removed; they are taken apart as B-samples. The residual parts of samples (A-samples; if any remaining) are mixed together, divided into 19 portions, each portion is coupled with a different Boc-amino acid.

Each of the 19 B-samples is treated as follows: the sample is divided into 19 portions then each portion is coupled with a different Boc-amino acid then the resultant 19x19 samples are submitted to the following inner cycle of operations until they attain the tetrapeptide size.

Inner cycle: each of the 19x19 samples is treated as follows: divided into 19 parts. Each part is coupled with one of the 19 Boc-amino acids then mixed together (end of both cycles).

The peptides of the 3x361 final samples are cleaved from the support according to Example 17 to obtain the kit of 1,2-, 2,3- and 3,4-types of second order vicinal sub-libraries.

Example 29

Second order vicinal sub-library sub-kit of a resin-bound normal tetrapeptide library

1.5 mmol samples of 10 different Boc-aminoacyl-GABA-aminomethyl resins (prepared from resin described in Example 4 according to Example 11; kinds of Boc-amino acid resin esters are listed in Example 25) are treated as described in Example 28 with the following modifications: 10 kinds of samples are used instead of 19; the number of final samples is 3x100 instead of 3x361; the final samples are treated as described in Example 16.

Example 30

Labeled second order vicinal sub-library sub-kit of a free normaltetrapeptide library synthesized on labeled supports

2.1 mmol samples of 9 kinds of color or fluorescence labeled resins (see Example 7) are submitted to 3 consecutive main cycles of operations.

Main cycle: 0.7 mmol of each resin sample is removed and taken apart as B-sample. The residual parts of samples (if any remaining) are named A-samples. Each of A-samples is treated as follows: the sample is divided into 9 portions, each portion is coupled with one of the 9 Boc-amino acids (Boc-amino acids listed in Example 25 are used with the following modifications: Boc-Pro is omitted; D-Phe is used instead of Phe). The lengthened samples are mixed together to form a new sample used as one of the starting samples in the next main cycle.

Each of B-samples is coupled with a different Boc-amino acid (see above), the 9 new samples are mixed together, divided into 9 portions, each portion is coupled with a different Boc-amino acid. The resultant 9 samples are consecutively submitted to the inner cycles of operations until they attain the tetrapeptide size.

Inner cycle: Each of the 9 samples (if the peptides are shorter than tetrapeptides) is treated as follows: the sample is divided into 9 portions and each portion is coupled with one of Boc-amino acids (see above) then mixed. The mixed samples (if shorter than tetrapeptides) are used as starting samples in the next inner cycle.

This is the end of both cycles.

The peptides of 3x9 final samples obtained are cleaved from the support according to Example 17.

Example 31

Second order vicinal sub-library sub-kit of a normal tetrapeptide library bound to color or fluorescent supports

2.1 mmol samples of 9 kinds of color or fluorescence labeled resins (see Example 5) are treated according to Example 30 with the exception of the last step in which the protecting groups are removed from the final samples as described in Example 16.

Example 32

Labeled resin-bound second order vicinal sub-library sub-kit of a normal tetrapeptide libra y (labeling in the course)

21 mmol of BGFL-aminomethyl resin beads (Example 6) is divided into 10 equal parts. Each part is coupled with one of 10 Boc-amino acids (Boc-amino acids listed in Example 25 are used with a single modification: Boc-L-α-aminobutyric acid is applied instead of Boc-Pro). The resultant samples are submitted to 3 consecutive main cycles of operations.

Main cycle: 0.7 mmol of each resin sample is removed and taken apart as one of the B-samples. The residual parts of samples (A-samples; if any remaining) are mixed together then divided into 10 parts and each of them is coupled with a different Boc-amino acid (see above). The resultant samples are used as starting samples in the next main cycle.

Each of B-samples is treated as follows: the Fmoc-group is removed according to Example 12 then the sample is coupled with a color or fluorescence marker (see Example 14). The resultant B-samples are mixed together and divided into 10 portions. Each of new portions is coupled with a different Boc-amino acid. The resultant 10 samples are consecutively submitted to the inner cycles of operations until they attain the tetrapeptide size.

Inner cycle: Each of the samples (if the peptides are shorter than tetrapeptides) is treated as follows: divided into 10 equal portions, the portions are coupled with different Boc-amino acids (see above) then mixed. The samples (if the peptides are shorter than tetrapeptides) are used as starting samples in the next inner cycle.

This is the end of both cycles.

The protecting groups of the 3x10 final samples are removed according to Example 16.

Example 33

Second order vicinal sub-library sub kit of a free normal tetrapeptide library carrying color or fluorescent labels at the C-termini of peptides (labeling in the course)

21 mmol of BFLG-hydroxymethyl resin (Example 8) is treated according to Example 32 with the exception of the

last step in which the peptides of the final samples are cleaved from the support as described in Example 17.

Example 34

Screening of 4A first order standard-C tetrapeptide sub-library (4A4), bound to unlabeled support, with rat red blood cells

The experiment is carried out as recently described by Sebestyen et al. (Bioorg. Med. Chem. Letters 1993, 3, 413-418). 0.5 mg of 4A4 and 0.5 mL of red blood cell suspension (prepared from heparinized rat blood; the cells are washed and centrifuged 3 times before use to remove serum proteins) is kept at 0°C for 15 min, shaking gently several times, then pipetted into 10 mL Seglen's suspension buffer (Ssb). The diluted suspension is gently centrifuged (200 rpm, 3 min) to facilitate sedimentation of the beads. The excess of cells is removed by carefully pipetting out the supernatant. The sediment is diluted with 3 mL Ssb, pipetted into a Petri-dish and surveyed in an inverted microscope.

Although most of the beads are bare, some are completely covered with cells so screening test of 4A4 is considered positive.

Example 35

Screening with a support-bound standard-C trapeptide amino acid tester sub-kit.

All of the 19 components of the support-bound standard-C tetrapeptide amino acid tester sub-kit are submitted to the screening test described in Example 34. All tests resulting in formation of at leas some completely covered beads are considered positive and the amino acid appearing in symbol of the component giving positive result is included into the amino acid occurrence list.

Example 36

The use of the components of an unlabeled resin-bound first sub-kit of a standard-C tetrapeptide library in screening:

To the amino acid occurrence list, A, E, H, K, L, N, Y, the following components of the first order sub-library sub-kit described in Example 20 are selected for screening described in Example 34: 1A, 2A, 3A, 4A, 1E, 2E, 3E, 4E, 1H, 2H, 3H, 4H, 1K, 2K, 3K, 4K, 1L, 2L, 3L, 4L, 1N, 2N, 3N, 4N, 1Y, 2Y, 3Y, 4Y. The amino acids appearing in the symbol of the components giving positive results are entered - from position to position - into the list of amino acids varied in the occurrence library.

Example 37

The use of the components of an unlabeled resin-bound second order standard C tetrapeptide sub-library sub-kit in screening:

In order to determine the sequences of the active peptides present in the occurrence library demonstrated in Table 6 the following components of the

Table 6.

| | |
|---|---|
| 1 | A |
| 2 | E, K |
| 3 | L, N |
| 4 | H, Y |

second order sub-library sub-kit described in Example 29 (or synthesized as the second order vicinal sub-library sub-kit of the occurrence library) are submitted to the screening test (Example 34): 2E3L, 2E3N, 2K3L, 2K3N, 3L4H, 3L4Y, 3N4H, 3N4Y. The active components may be used as dominoes in determining the sequences of the active peptides present in the occurrence library as shown below.

```
1A
  2E3L
    3L4H
                                  C 1  4
The deduced sequence is:           AELH

Written in usual order:            HLEA
```

In some cases testing with disjunct second order sub-libraries is also needed.

Example 38

First sub-kit of a free general tripeptide library

/i/ 1.0 mmol samples of each of Boc-amino acid resins derived from Ala, Asn, Gly, net, Pro and Val are used as starting materials. One-third of each of 6 samples is removed and taken apart (B1-samples).
/ii/ The residual parts of each sample (Al-samples) are mixed then portioned into 4 equal parts. Each part is coupled with one of the following Boc-amino acids: Boc-Glu(OBzl), Boc-Leu, Boc(Z)-Lys and Boc-Phe.
/iii/ Half of each of the resultant 4 samples (see paragraph /ii/) is removed and taken apart (B2-samples). The residual parts of each sample (A2-samples) are mixed then divided into 10 equal parts. Each part is coupled with one of the Boc-amino acids listed in Example 25 to produce 10 final samples.
/iv/ Each B1-sample (see paragraph /i/) is treated as follows: divided into 4 equal parts and each part is coupled with one of Boc-amino acids listed in paragraph /ii/ then mixed.
/v/ Each of the resultant 4 samples (see paragraph /iv/) is treated as follows: divided into 10 equal parts and each part is coupled with one of Boc-amino acids listed in Example 25 then mixed.
/vi/ Each B2-sample (see paragraph /iii/) is treated as described in paragraph /v/.
/vii/ The 3x10 final samples obtained in paragraphs /iii/, /v/ and /vi/ are treated according to Example 17.

Example 39

Labeled second order 1,3 and 2,4 type disjunct sub-library sub-kit of a resin-bound normal tetrapeptide library. The labels refer to the lower non-varied coupling position (labeling in the course)

20 mmol of BGFL-aminomethyl resin beads (Example 6) is divided into 10 equal parts. Each part is coupled with one of 10 Boc-amino acids (Boc-amino acids listed in Example 25 are used with a single modification: Boc-L-α-aminobutyric acid is applied instead of Boc-Pro). The resultant samples are submitted to 2 consecutive main cycles of operations.

Main cycle: 1.0 mmol of each resin sample is removed and taken apart as one of the B-samples. The residual parts of samples (A-samples; if any remaining) are mixed together then divided into 10 parts and each of them is coupled with a different Boc-amino acid (see above). The resultant samples are used as starting samples in the next main cycle.

Each of B-samples is treated as follows: the Fmoc-group is removed according to Example 12 then the sample is coupled with a color or fluorescence marker (see Example 14). The resultant 10 samples are consecutively submitted to two inner cycles of operations.

Inner cycle: The resultant B-samples are mixed together and divided into 10 portions. Each of new portions is coupled with a different Boc-amino acid. This is the end of main cycle and the inner cycle.

Each of the samples coming out from the second inner cycle (if the peptides are shorter than tetrapeptides) is treated as follows: divided into 10 equal portions, the portions are coupled with different Boc-amino acids (see above) then mixed. The protecting groups of the 2x10 final samples are removed according to Example 16.

## Claims

1. A kit of peptide mixtures for determination of the amino acid sequences of bioactive peptides in a parent peptide library comprising

   a "first sub-kit" which consists of a complete set of first order sub-libraries of said parent peptide library and
   a "second sub-kit" of peptide mixtures consisting of a complete set of vicinal and disjunct type second order and possibly higher order sub-libraries of the same parent peptide library or a partial library (occurrence library) of the same parent peptide library
   and possibly also comprising an amino acid tester sub-kit ("third sub-kit"), consisting of combined components of the first sub-kit.

2. A kit according to claim 1 wherein the components of the first sub-kit are special partial libraries of the parent peptide library in which the same amino acid occupies a certain position in all peptides while in the rest of positions the amino acids are varied like in the parent peptide library.

3. A kit according to claim 1 wherein the components of the second sub-kit are special partial libraries of the parent peptide library in which the same two positions are occupied by the same two amino acids in all peptides while in the rest of positions the amino acids are varied like in the parent peptide library.

4. A kit according to claim 1 wherein the components of the amino acid tester kit sub-kit are combined mixtures of all those components of the first sub-kit in which the non-varied position is occupied by the same amino acid.

5. A kit according to any of claims 1 to 4 in which the number of amino acid residues of the peptides of parent peptide libraries is in the range of 2 to 25.

6. A kit according to any of claims 1 to 5 in which the amino acid subunits of peptides are L-amino acids, D-amino acids, unusual amino acids and/or derivatives of amino acids.

7. A kit according to any of claims 1 to 6 comprising mixtures of free peptides or peptides bound to labeled solid support.

8. A kit of free peptides according to any of claims 1 to 7 in which color and/or fluorescent marker groups are attached to the C- and/or N-termini of peptides.

9. A kit according to any of claims 1 to 7 in which the components of the kits are mixtures of sub-libraries attached to solid supports differing in color and/or fluorescence and/or specific gravity and/or the size of the beads, where the color and/or the fluorescence and/or the specific gravity and/or the size of the beads refer to (i) the coupling position of the non-varied amino acid, (ii) the amino acid occupying the non-varied position (iii) both the non-varied amino acid and its position, thus making the mixed sub-libraries distinguishable.

10. Process for the synthesis of peptide sub-library kits according to any of claims 1 to 9 which comprises combination and/or repetition of at least one of the following operations;

    (i) portioning into *p* equal portions of a solid support or an amino acylated support or a mixed amino acylated support or a support carrying peptides or a support carrying a mixture of peptides where *p* is the number of amino acids varied in the parent peptide library in a given coupling position;
    (ii) each of *p* portions are submitted to removal of the terminal amino-protecting group and coupling with a different protected amino acid;
    (iii) mixing *p* portions;
    (iv) removal of $1/(n\text{-}r\text{+}1)$ part of each of *p* portions giving samples B, and the residues which are named (if any remaining) samples A, where *r* and *n* are the number of amino acid residues already attached to the support and the number of amino acid residues in the peptides of the parent peptide library, respectively:
    (v) execution of operations (i) to (iii) on each of *p* samples;
    (vi) execution of operations (i)-(ii) on each of *p* portions;
    (vii) two sets of B-samples, each set comprising *p* samples, are combined in pairs containing the same amino acid residue in the non-varied position of the peptides to form *p* combined samples, named B-samples;
    (viii) coupling a different protected amino acid to each of *p* portions;

(ix) execution of operations (i) to (iii) on one sample;

(x) submission of each of *p* portions to removal of $R^2$ protecting group of the support and to coupling with a different color or fluorescent marker;

(xi) removal of terminal amino-protecting group and coupling a different color or fluorescent marker to each of *p* portions;

(xii) portioning of each of *p* equal samples into *p* equal portions;

(xiii) removal of 1/(*n-r*) part of each of *p* portions otherwise the same as operation (iv);

(xiv) submission of each of *p* portions to removal of $R^2$ protecting group of the support and to coupling with a different color or fluorescent marker;

(xv) removal of 1/(*n-r*-1) part of each of *p* portions, otherwise the same as operation (iv).

**11.** Process for the synthesis of first sub-kit according to any of claim 10 which comprises unlabeled free peptides in which the process is started with *p* equal samples of N-protected O-aminoacyl support, all samples differing in the aminoacyl residue;

(i) first, the samples are submitted to operation (iv) of claim 10, then samples A are consecutively submitted to operations (iii), (i) and (ii) of claim 10 and the resultant mixtures are used as starting samples in the next cycle of operations;

(ii) samples B are submitted (*n-r*) times to operation (v) of claim 10 then the samples are taken apart:

(iii) the cycle of operations (i) to (ii) of this claim is repeated (*n*-2) times;

(iv) all samples are submitted to deprotection and cleavage from the support.

**12.** A process according to claim 10 for preparing a first sub-kit of free peptides carrying color or fluorescent labels at their C-terminus assigned to the amino acid residues occupying the non-varied positions;

(i) the process is started with *p* equal samples of solid supports comprising N-protected O-aminoacyl groups wherein the aminoacyl grop is a color or fluorescent marker differing in color or fluorescence of the label of the support;

(ii) the samples are submitted to operation (xiii) of claim 10;

(iii) samples A (if any remaining) are submitted to operation (v) of claim 10 then are used as starting samples in the next cycle of operations;

(iv) samples B are submitted to operation (ii) of claim 10, then mixed and submitted consecutively (*n-r*) times to operation (ix) of claim 10 then the sample is taken apart;

(v) operations (ii) to (iv) of this claim are repeated (*n*-1) times;

(vi) all samples are submitted to deprotection and cleavage from the support.

**13.** A process for synthesizing a first sub-kit of free peptides carrying color or fluorescent labels at their C-terminus assigned to the amino acid residues occupying the non-varied positions, in which the process is started with one sample of solid support comprising O-acylated hydroxymethyl groups corresponding to the following general formula where $R^1$ and $R^2$ are protecting groups, $_j$, $_k$, and $_f$ are integers 1 to 6;

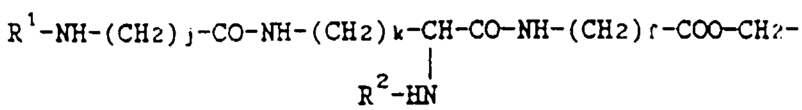

(i) the sample is submitted to operations (i) and (ii) of claim 10;

(ii) the samples are then submitted to operation (iv) of claim 10;

(iii) samples B are submitted to operation (x) of claim 10, then the samples are mixed and submitted (*n-r*) times to operation (ix) of claim 10 and the sample is taken apart;

(iv) samples A (if any remaining) are mixed then submitted to operations (i) and (ii) of claim 10, then are used as starting samples in the next cycle of operations;

(v) The cycle of operations (ii) to (iv) of this claim is repeated (*n*-1) times;

(vi) all samples are submitted to deprotection and cleavage from the support.

**14.** A process for synthesizing a first sub-kit of free peptides carrying color or fluorescent labels at their N-terminus assigned to the amino acid residues occupying the non-varied positions, in which the process is started with *p*

equal samples of N-protected O-aminoacyl support, all samples differing in the aminoacyl residue;

(i) first, the samples are submitted to operation (iv) of claim 10;
(ii) samples B are (*n-r*) times consecutively submitted to operation (v) of claim 10, then to one operation (xi) of claim 10, and after mixing, the sample is taken apart;
(iii) samples A (if any remaining) are consecutively submitted to operations (iii), (i) and (ii) of claim 10 and the resultant mixtures are used as starting samples in the next cycle of operations;
(iv) the cycle of operations (i) to (iii) of this claim is repeated (*n*-1) times;
(v) all samples are submitted to deprotection and cleavage from the support.

**15.** A process for synthesizing an amino acid tester sub-kit of free peptides comprising the following steps:

(i) the process is started with *p* equal samples of N-protected O-aminoacyl support, all samples differing in the acylamino residue;
(ii) first, the samples are submitted to operation (iv) of claim 10;
(iii) on samples A (if any remaining) operations (iii), (i) and (ii) of claim 10 are consecutively executed then the samples are used in the next cycle operations;
(iv) samples B together with the resultant B samples of the previous cycle (if executed) are submitted to operation (vii) of claim 10;
(v) if $r < n$ samples B are submitted to one operation (v) of claim 10 otherwise are taken apart;
(vi) cycle comprising operations (ii) to (v) of this claim are repeated (*n*-1) times;
(vii) finally, the resultant B samples are submitted to deprotection and cleavage from the support.

**16.** A process for synthesizing an amino acid tester sub-kit of peptides bound to unlabeled support comprising the following steps:

(i) the process is started with a sample of solid support, carrying blocked primary amino groups attached through a spacer;
(ii) The sample is first consecutively submitted to deprotection and operations (i) and (viii) of claim 10;
(iii) then the samples are treated according to operations (ii) to (vi) of claim 16;
(vi) finally the resultant B samples are submitted to deprotection.

**17.** A process for synthesizing a first sub-kit of a normal parent peptide library bound to color and/or fluorescent support where the color, or the fluorescence, or the size or the specific gravity of the beads or combinations of these characteristics are assigned to the amino acid residues occupying the non-varied position;

(i) the process is started with *p* equal samples of polymer and glass supports of different bead sizes comprising free or protected primary amino groups wherein the beads are colored or fluorescent, differing in at least one of the mentioned characteristics;
(ii) the samples are submitted to operations (ii) to (v) of claim 13;
(iii) finally all samples are submitted to deprotection.

**18.** A process for synthesizing a first sub-kit bound to color and/or fluorescent supports, using solid support comprising acylaminoalkyl groups corresponding to the following general formula where $R^1$ and $R^2$ are protecting groups, $_j$, $_k$, and $_f$ are integers 1 to 6

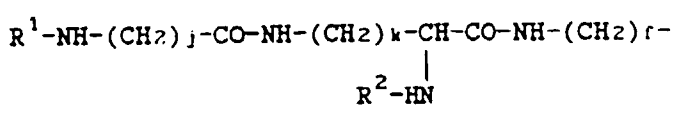

and the color or fluorescent markers as signed to the amino acid residues occupying the non-varied position are attached to the support in the course of the peptide synthesis;

(i) the synthesis is started with one sample of support;
(ii) the sample is submitted to operations (i) to (v) of claim 14;
(iii) finally, all samples are submitted to deprotection.

**19.** A process for synthesizing a sub-kit comprising all vicinal type second order sub-libraries of a parent peptide library of unlabeled soluble peptides, in which the process is started with *p* equal samples of O-acylamino support, all samples differing in the acylamino residue;

(i) the samples are submitted to operation (xiii) of claim 10;
(ii) samples A (if any remaining) are consecutively submitted to operations (iii), (i), (ii) of claim 10 then the resultant A samples are used in the next cycle of operations;
(iii) samples B are submitted to operation (vi) of claim 10 then the unmixed resultant samples are one by one submitted to *n-r* consecutive cycles of operations (i) to (iii) of claim 10;
(iv) all resultant samples originating from samples B are taken apart;
(v) cycle of operations (i) to (iv) this claim are repeated *n*-2 times;
(vi) all samples are submitted to cleavage and deprotection.

**20.** A process for synthesizing a sub-kit bound to unlabeled support comprising all vicinal type second order sub-libraries of a parent peptide library, in which a sample of solid support, carrying blocked primary amino groups attached through a spacer, is consecutively submitted to deprotection, operations (i) and (viii) of claim 10, then the samples are treated according operations (i) to (v) of claim 19, and finally all samples are submitted to deprotection.

**21.** A process for synthesizing a sub-kit comprising all vicinal type second order sub-libraries of a parent peptide library of soluble peptides, carrying color or fluorescent labels at their C-terminus, assigned to the amino acid residue occupying the lower coupling position of the two vicinal non-varied positions, in which the process is started with *p* equal samples of solid support comprising N-protected O-aminoacyl groups wherein the aminoacyl group is a color or fluorescent marker, differing in color or fluorescence of the label of the support;

(i) first, operation (xv) of claim 10 is executed;
(ii) samples A (if any remaining) are submitted to operation (v) of claim 10, then the resultant A samples are used in the next cycle of operations;
(iii) samples B are consecutively submitted to operations (ii), (iii), (i) and (ii) of claim 10, then the resultant samples are submitted to *n-r* consecutive cycles of operation (v) of claim 10 then the samples are taken apart;
(iv) cycle of operations (i) to (iii) of this claim is repeated (*n-2*) times;
(v) all samples are submitted to cleavage and deprotection.

**22.** A process for synthesizing a sub-kit comprising all vicinal type second order sub-libraries of a normal parent library of peptides bound to color or fluorescent supports, when the color or fluorescence or the size or the specific gravity of the support is assigned to the amino acid residue occupying the lower coupling position of the two vicinal non-varied positions, and in which the process is started with *p* equal samples of polymer and glass supports comprising free or protected primary amino groups, wherein the beads of different size are colored or fluorescent, differing in at least one of the mentioned characteristics of the support, then the samples are submitted to operations (i) to (iv) of claim 21, finally all samples are submitted to deprotection.

**23.** A process for synthesizing a sub-kit comprising all vicinal type second order sub-libraries of a normal parent library of peptides bound to color or fluorescent supports, when the color or fluorescence of the support is assigned to the amino acid residue occupying the lower coupling position of the two vicinal non-varied positions, and in which the process is started with a single deprotected sample of solid support comprising acylaminoalkyl groups corresponding to the following general formula where $R^1$ and $R^2$ are protecting groups, $_j$, $_k$, and $_f$ are integers 1 to 6

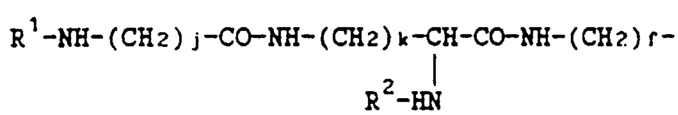

and the color or fluorescent labels are attached to the support in the course of the peptide synthesis;

(i) the sample is submitted to operations (i) and (ii) of claim 10;
(ii) the samples are submitted to operation (xiii) of claim 10;
(iii) samples A (if any remaining) are consecutively submitted to operations (iii), (i), (ii) of claim 10 then the

resultant A samples are used in the next cycle of operations;
(iv) samples B are submitted to operation (x) of claim 10, then mixed and submitted to operations (i) and (ii) of claim 10 then the resultant samples are submitted to *n-r* consecutive cycles of operation (v) of claim 10 then the samples are taken apart;
(v) cycle of operations (ii) to (iv) of this claim is repeated *n-2* times;
(vi) all samples are submitted to deprotection.

**24.** A method for determination of the amino acid sequences of bioactive peptides ("domino strategy") using kits according to claims 1 to 9 whereby the use of unlabeled sub-kits comprises three consecutive steps;

(i) determination of the amino acid occurrence list by executing the screening experiments with all components of the amino acid tester sub-kit;
(ii) determination of the occurrence library by carrying out screening experiments with selected components of the first sub-kit;
(iii) determination of the amino acid sequences by the domino like use in screening of selected components of the second sub-kit(s) of the parent peptide library or by synthesizing the second sub-kit of the occurrence library and doing screening tests with its components;

while when using labelled kits the experiments described in (i) can be omitted, those of (ii) are executed with all components of the first sub-kit, and (iii) is applied unchanged.

## Patentansprüche

**1.** Peptidgemisch-Kit bzw. Testsatz zur Bestimmung von Aminosäuresequenzen von bioaktiven Peptiden in einer Stammpeptidbibliothek, umfassend

einen "ersten Subtestsatz", der aus einem vollständigen Satz von Subbibliotheken erster Ordnung der Stammpeptidbibliothek und

einen "zweiten Subtestsatz" von Peptidgemischen, bestehend aus einem vollständigen Satz von Subbibliotheken zweiter Ordnung und möglicherweise höherer Ordnung vom vicinalen und disjunkten Typ der gleichen Stammpeptidbibliothek oder aus einer Teilbibliothek (Ereignis-Bibliothek) der gleichen Stammpeptidbibliothek, und möglicherweise ebenfalls umfassend einen Aminosäuretestsubstanz-Subtestsatz ("dritter Testsatz"), bestehend aus kombinierten Komponenten des ersten Subtestsatzes.

**2.** Testsatz nach Anspruch 1, wobei die Komponenten des ersten Subtestsatzes spezielle Teilbibliotheken der Stammpeptidbibliothek sind, in denen die gleiche Aminosäure eine bestimmte Position in allen Peptiden besetzt, während in den restlichen Positionen die Aminosäuren wie in der Stammpeptidbibliothek unterschiedlich sind.

**3.** Testsatz nach Anspruch 1, wobei die Komponenten des zweiten Subtestsatzes spezielle Teilbibliotheken der Stammpeptidbibliothek sind, in welchen die gleichen zwei Positionen durch die gleichen zwei Aminosäuren in allen Peptiden besetzt sind, während in den restlichen Positionen die Aminosäuren wie in der Peptidstammbibliothek unterschiedlich sind.

**4.** Testsatz nach Anspruch 1, wobei die Komponenten des Aminosäuretestsubstanz-Subtestsatzes kombinierte Gemische all dieser Komponenten des ersten Subtestsatzes sind, in welchem die nicht-unterschiedliche Position durch die gleiche Aminosäure besetzt ist.

**5.** Testsatz nach einem der Ansprüche 1 bis 4, in welchem die Anzahl der Aminosäurereste der Peptide der Stammpeptidbibliotheken in dem Bereich von 2 bis 25 ist.

**6.** Testsatz nach einem der Ansprüche 1 bis 5, in dem die Aminosäure-Untereinheiten der Peptide L-Aminosäuren, D-Aminosäuren, ungewöhnliche Aminosäuren und/oder Aminosäurederivate sind.

**7.** Testsatz nach einem der Ansprüche 1 bis 6, umfassend Gemische von freien Peptiden oder Peptiden, die an einen markierten, festen Träger gebunden sind.

**8.** Testsatz von freien Peptiden nach einem der Ansprüche 1 bis 7, in welchem farbige und/oder fluoreszierende Markierungsgruppen an die C-und/oder N-Termini der Peptide geknüpft sind.

**9.** Testsatz nach einem der Ansprüche 1 bis 7, in welchem die Komponenten der Testsätze Gemische von Subbibliotheken sind, die an feste Träger geknüpft sind, die sich in Farbe und/oder Fluoreszenz und/oder spezifischem Gewicht und/oder Größe unterscheiden, wobei die Farbe und/oder die Fluoreszenz und/oder das spezifische Gewicht und/oder die Größe der Partikel sich auf (i) die Kupplungsposition der nicht-unterschiedlichen Aminosäure, (ii) auf die Aminosäure, welche die nicht-unterschiedliche Position besetzt, (iii) sowohl auf die nicht-unterschiedliche Aminosäure als auch ihre Position bezieht, um damit die gemischten Subbibliotheken unterscheidbar zu machen.

**10.** Verfahren zur Synthese von Peptid-Subbibliothek-Testsätzen nach einem der Ansprüche 1 bis 9, welches Kombination und/oder Wiederholung von mindestens einem der folgenden Operationen umfaßt;

(i) Portionieren in p äquivalente Portionen eines festen Trägers oder eines aminoacylierten Trägers oder eines gemischten aminoacylierten Trägers oder eines Trägers, der Peptide trägt, oder eines Trägers, der ein Gemisch von Peptiden trägt, wobei p die Anzahl der Aminosäuren ist, unterschiedlich in der Stammmpeptidbibliothek in einer gegebenen Kupplungsposition;
(ii) jede der p Portionen wird der Entfernung der terminalen, Amino-Schutzgruppe und der Kupplung mit einer verschiedenen, geschützten Aminosäure unterworfen;
(iii) Mischen von p Portionen;
(iv) Beseitigung von 1/(n-r+1) Teilen von jeder der p Portionen, was Proben B ergibt, und die Reste, die Proben A (sofern welche übrig bleiben) genannt werden, wobei r und n die Anzahl der Aminosäurereste, die schon an den Träger angeknüpft sind, bzw. die Anzahl der Aminosäurereste in den Peptiden der Peptidstammbibliothek sind;
(v) Ausführen der Operationen (i) bis (iii) an jeder der p Proben;
(vi) Ausführen der Operationen (i) - (ii) an jeder von p Portionen;
(vii) zwei Sätze von B-Proben, wobei jeder Satz p Proben umfaßt, sind in Paaren kombiniert, die den gleichen Aminosäurerest in der nicht-unterschiedlichen Position der Peptide enthalten, um p kombinierte Proben zu bilden, genannt B-Proben;
(viii) Kuppeln einer verschiedenen, geschützten Aminosäure mit jeder der p Portionen;
(ix) Ausführen der Operationen (i) bis (iii) an einer Probe;
(x) Unterwerfen jeder der p Portionen der Beseitigung der $R^2$ Schutzgruppe des Trägers und Kuppeln mit einem verschiedenen, farbigen oder fluoreszierenden Markierungsmittel;
(xi) Entfernen der terminalen Amino-Schutzgruppe und Kuppeln eines verschiedenen, farbigen oder fluoreszierenden Markierungsmittel mit jeder der p Portionen;
(xii) Portionieren jeder der p gleichen Proben in p gleiche Portionen;
(xiii) Entfernen von 1/(n-r) Teilen von jeder der p Portionen, sonst das gleiche wie Operation (iv);
(xiv) Unterwerfen jeder der p Portionen dem Entfernen einer $R^2$ Schutzgruppe des Trägers und Kuppeln mit einem verschiedenen, farbigen oder fluoreszierenden Markierungsmittel;
(xv) Entfernen von 1/(n-r-1) Teilen von jeder der p Portionen, sonst das gleiche wie Operation (iv).

**11.** Verfahren zur Synthese des ersten Subtestsatzes nach Anspruch 10, das unmarkierte, freie Peptide umfaßt, in welchem das Verfahren mit p gleichen Proben eines N-geschützten O-Aminoacyl-Trägers gestartet wird, wobei sich alle Proben in dem Aminoacylrest unterscheiden;

(i) die Proben werden zunächst Operation (iv) von Anspruch 10 unterworfen, dann werden die Proben A nacheinander den Operationen (iii), (i) und (ii) von Anspruch 10 unterworfen und die resultierenden Gemische werden als Startproben in dem nächsten Operationszyklus verwendet;
(ii) Proben B werden (n-r) mal Operation (v) von Anspruch 10 unterworfen, dann werden die Proben nacheinander analysiert;
(iii) der Operationszyklus (i) bis (ii) dieses Anspruchs wird (n-2) Mal wiederholt;
(iv) alle Proben werden einem Entschützen und Abspalten von dem Träger unterzogen.

**12.** Verfahren nach Anspruch 10 zur Herstellung eines ersten Subtestsatzes von freien Peptiden, die farbige oder fluoreszierende Markierungen an ihrem C-Terminus tragen, die den Aminosäureresten zugeteilt sind, welche die nicht-unterschiedlichen Positionen besetzen;

(i) das Verfahren wird mit p gleichen Proben fester Träger gestartet, die N-geschützte O-Aminoacylgruppen umfassen, wobei die Aminoacylgrppe ein farbiges oder fluoreszierendes Markierungsmittel ist, das sich in Farbe oder Fluoreszenz von der Markierung des Trägers unterscheidet;
(ii) die Proben werden der Operation (xiii) von Anspruch 10 unterworfen;
(iii) Proben A (sofern welche übrig bleiben) werden Operation (v) von Anspruch 10 unterworfen, anschließend werden sie als Startproben in dem nächsten Operationszyklus verwendet;
(iv) Proben B werden der Operation (ii) von Anspruch 10 unterworfen, dann gemischt und nacheinander (n-r) mal Operation (ix) von Anspruch 10 unterworfen, dann werden die Proben nacheineinander analysiert;
(v) Operationen (ii) bis (iv) dieses Anspruches werden (n-1) mal wiederholt;
(vi) alle Proben werden einem Entschützen und Abspalten von dem Träger unterworfen.

**13.** Verfahren zur Synthese eines ersten Subtestsatzes von freien Peptiden, die farbige oder fluoreszierende Markierungen an ihrem C-Terminus tragen, die den Aminosäureresten zugeordnet sind, welche die nicht-unterschiedlichen Positionen besetzen, wobei das Verfahren mit einer Probe des festen Trägers gestartet wird, der O-acylierte Hydroxymethylgruppen umfaßt im Einklang mit der folgenden, allgemeinen Formel, worin $R^1$ und $R^2$ Schutzgruppen sind, j, k und f ganze Zahlen von 1 bis 6 sind;

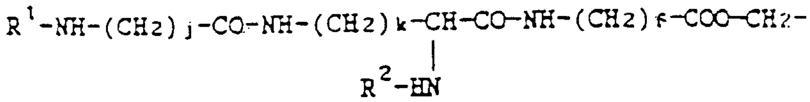

(i) die Probe wird den Operationen (i) und (ii) von Anspruch 10 unterworfen;
(ii) die Proben werden anschließend der Operation (iv) von Anspruch 10 unterworfen;
(iii) Proben B werden der Operation (x) von Anspruch 10 unterworfen, anschließend werden die Proben gemischt und (n-r) mal Operation (ix) von Anspruch 10 unterworfen, und die Proben werden nacheineinander analysiert;
(iv) Proben A (sofern welche übrigbleiben) werden gemischt, anschließend den Operationen (i) und (ii) von Anspruch 10 unterworfen, anschließend werden sie als Startproben in dem nächsten Operationszyklus verwendet;
(v) der Operationszyklus (ii) bis (iv) dieses Anspruches wird (n-1) mal wiederholt;
(vi) alle Proben werden einem Entschützen und Abspalten von dem Träger unterworfen.

**14.** Verfahren zur Synthese eines ersten Subtestsatzes von freien Peptiden, die farbige oder fluoreszierende Markierungsmittel an ihrem N-Terminus tragen, die den Aminosäureresten zugeordnet sind, welche die nicht-unterschiedlichen Positionen besetzen, wobei das Verfahren mit p gleichen Proben eines N-geschützten, O-Aminoacyl-Trägers gestartet wird, wobei alle Proben sich in dem Aminoacylrest unterscheiden;

(i) die Proben werden zunächst der Operation (iv) von Anspruch 10 unterworfen;
(ii) Proben B werden (n-r) mal nacheinander der Operation (v) von Anspruch 10 unterworfen, dann der Operation (xi) von Anspruch 10, und nach Mischen wird die Probe nacheineinander analysiert;
(iii) Proben A (sofern welche übrigbleiben) werden nacheinander den Operationen (iii), (i) und (ii) von Anspruch 10 unterworfen und die resultierenden Gemische werden als Startproben in dem nächsten Operationszyklus verwendet;
(iv) der Operationszyklus (i) bis (iii) dieses Anspruches wird (n-1) mal wiederholt;
(v) alle Proben werden einem Entschützen und Abspalten von dem Träger unterzogen.

**15.** Verfahren zur Synthese eines Aminosäuretestsubstanz-Subtestsatzes von freien Peptiden, umfassend die folgenden Schritte:

(i) das Verfahren wird mit p gleichen Proben eines N-geschützten O-Aminoacyl-Trägers gestartet, wobei sich alle Proben in dem Acylaminorest unterscheiden;
(ii) die Proben werden zunächst der Operation (iv) von Anspruch 10 unterworfen;
(iii) die Operationen (iii), (i) und (ii) von Anspruch 10 werden nacheinander an den Proben A (sofern welche übrigbleiben) ausgeführt, dann werden die Proben in dem nächsten Operationszyklus verwendet;
(iv) Proben B werden zusammen mit den resultierenden B-Proben des vorherigen Zyklus (sofern ausgeführt) der Operation (vii) von Anspruch 10 unterworfen;

(v) wenn r < n, werden Proben B der Operation (v) von Anspruch 10 unterworfen, sonst werden sie nacheineinander analysiert;
(vi) der Zyklus, der die Operationen (ii) bis (v) dieses Anspruches umfaßt, wird (n-1) mal wiederholt;
(vii) schließlich werden die resultierenden B-Proben einem Entschützen und Abspalten von dem Träger unterworfen.

**16.** Verfahren zur Synthese eines Aminosäuretestsubstanz-Subtestsatzes von Peptiden, die an einen unmarkierten Träger gebunden sind, umfassend die folgenden Schritte:

(i) das Verfahren wird mit einer Probe eines festen Trägers gestartet, der blockierte primäre Aminogruppen, die durch einen Spacer verknüpft sind, trägt;
(ii) die Probe wird zunächst hintereinander einem Entschützen und Operationen (i) und (viii) von Anspruch 10 unterworfen;
(iii) die Proben werden anschließend gemäß den Operationen (ii) bis (vi) von Anspruch 16 behandelt;
(vi) schließlich werden die resultierenden B-Proben einem Entschützen unterworfen.

**17.** Verfahren zur Synthese eines ersten Subtestsatzes einer normalen Stammpeptidbibliothek, gebunden an einen farbigen und/oder fluoreszierenden Träger, wobei die Farbe, oder die Fluoreszenz oder die Größe oder das spezifische Gewicht der Partikel oder Kombinationen dieser Charakteristika den Aminosäureresten zugeordnet sind, die die nicht-unterschiedliche Position besetzen;

(i) das Verfahren wird mit p gleichen Polymerproben gestartet und Glasträgern verschiedener Partikelgrößen, umfassend freie oder geschützte, primäre Aminogruppen, wobei die Partikel gefärbt oder fluoreszierend sind, unterschiedlich in mindestens einer der genannten Charakteristika ;
(ii) die Proben Operationen (ii) bis (v) von Anspruch 13 unterworfen;
(iii) schließlich werden alle Proben einem Entschützen unterworfen.

**18.** Verfahren zur Synthese eines ersten Subtestsatzes, gebunden an farbige und/oder fluoreszierende Träger, unter Verwendung eines festen Trägers, der Acylaminoalkylgruppen umfaßt im Einklang mit der folgenden, allgemeinen Formel, worin $R^1$ und $R^2$ Schutzgruppen sind, j, k und f ganze Zahlen von 1 bis 6

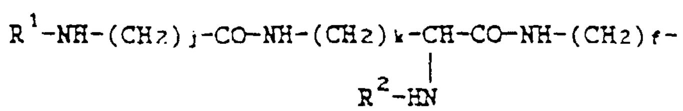

und die farbigen oder fluoreszierenden Markierungsmittel, die den Aminosäureresten zugeordnet sind, welche die nicht-unterschiedliche Position besetzen, an den Träger angeknüpft sind im Zuge der Peptidsynthese;

(i) die Synthese wird mit einer Probe des Trägers gestartet;
(ii) die Probe wird den Operationen (i) bis (v) von Anspruch 14 unterworfen;
(iii) abschließend werden alle Proben einem Entschützen unterworfen.

**19.** Verfahren zur Synthese eines Subtestsatzes, umfassend alle Subbibliotheken zweiter Ordnung vom vicinalen Typ einer Stammpeptidbibliothek von unmarkierten, löslichen Peptiden, in welchem das Verfahren mit p gleichen Proben eines O-Acylamino-Trägers gestartet wird, wobei alle Proben sich in dem Acylaminorest unterscheiden;

(i) die Proben werden der Operation (xiii) von Anspruch 10 unterworfen;
(ii) Proben A (sofern welche übrigbleiben) werden nacheinander den Operationen (iii), (i), (ii) von Anspruch 10 unterworfen, dann werden die resultierenden A-Proben in dem nächsten Operationszyklus verwendet;
(iii) Proben A werden der Operation (vi) von Anspruch 10 unterworfen, dann werden die nicht gemischten, resultierenden Proben eine nach der anderen n-r aufeinanderfolgenden Operationszyklen (i) bis (iii) von Anspruch 10 unterworfen;
(iv) alle resultierenden Proben, die von Proben B abstammen, werden nacheineinander analysiert;
(v) die Operationszyklen (i) bis (iv) dieses Anspruches werden n-2 mal wiederholt;
(vi) alle Proben werden einem Abspalten und Entschützen unterworfen.

**20.** Verfahren zur Synthese eines Subtestsatzes, gebunden an einen nicht markierten Träger, umfassend alle Subbibliotheken zweiter Ordnung vom vicinalen Typ einer Stammpeptidbibliothek, in welchem eine Probe des festen Trägers, der blockierte, durch einen Spacer angeknüpfte, primäre Aminogruppen trägt, nacheinander einem Entschützen, den Operationen (i) und (viii) von Anspruch 10 unterworfen werden, und anschließend die Proben gemäß den Operationen (i) bis (v) von Anspruch 19 behandelt werden und letztlich alle Proben einem Entschützen unterworfen werden.

**21.** Verfahren zur Synthese eines Subtestsatzes, umfassend alle Subbibliotheken zweiter Ordnung vom vicinalen Typ einer Stammpeptidbibliothek von löslichen Peptiden, die farbige oder fluoreszierende Markierungen an ihrem C-Terminus tragen, welche den Aminosäureresten zugeordnet sind, welche die niedrigere Kupplungsposition der zwei vicinalen nicht-unterschiedlichen Positionen besetzen, wobei das Verfahren gestartet wird mit p gleichen Proben eines festen Trägers, der N-geschützte, O-Aminoacylgruppen umfaßt, worin die Aminoacylgruppe ein farbiges oder fluoreszierendes Markierungsmittel ist, die sich in Farbe und Fluoreszenz von der Markierung des Trägers unterscheidet;

(i) zunächst wird Operation (xv) von Anspruch 10 ausgeführt;
(ii) Proben A (sofern welche übrigbleiben) werden Operation (v) von Anspruch 10 unterworfen, dann werden die resultierenden A-Proben in dem nächsten Operationszyklus verwendet;
(iii) Proben B werden nacheinander den Operationen (ii), (iii), (i) und (ii) unterworfen, dann werden die resultierenden Proben n-r aufeinanderfolgenden Operationszyklen (5) von Anspruch 10 unterworfen, dann werden die Proben nacheineinander analysiert;
(iv) Operationszyklus (i) bis (iii) dieses Anspruches wird (n-2) mal wiederholt;
(v) alle Proben werden einem Abspalten und Entschützen unterworfen.

**22.** Verfahren zur Synthese eines Subtestsatzes, umfassend alle Subbibliotheken zweiter Ordnung vom vicinalen Typ einer normalen Stammbibliothek von Peptiden, gebunden an farbige oder fluoreszierende Träger, wenn die Farbe oder Fluoreszenz oder die Größe oder das spezifische Gewicht des Trägers dem Aminosäurerest zugeordnet ist, der die niedrigere Kupplungsposition der zwei vicinalen, nicht-unterschiedlichen Positionen besetzt, und in welchem das Verfahren mit p gleichen Polymerproben gestartet wird und Glasträgern, die freie oder geschützte, primäre Aminogruppen umfassen, wobei die Partikel verschiedener Größe gefärbt oder fluoreszierend sind, sich in mindestens einer der genannten Charakteristika des Trägers unterscheiden, wobei anschließend die Proben den Operationen (i) bis (iv) von Anspruch 21 unterworfen werden, und schließlich alle Proben einem Entschützen unterworfen werden.

**23.** Verfahren zur Synthese eines Subtestsatzes, umfassend alle Subbibliotheken zweiter Ordnung vom vicinalen Typ einer normalen Stammbibliothek von Peptiden, gebunden an farbige oder fluoreszierende Träger, wenn die Farbe oder Fluoreszenz des Trägers dem Aminosäurerest zugeordnet ist, welcher die niedrigere Kupplungsposition der zwei vicinalen, nicht-unterschiedlichen Positionen besetzt, und in welchem das Verfahren mit einer einzigen, entschützten Probe eines festen Trägers gestartet wird, der Acylaminoalkylgruppen umfaßt im Einklang mit der folgenden allgemeinen Formel, wobei $R^1$ und $R^2$ Schutzgruppen sind, j, k und f ganze Zahlen von 1 bis 6

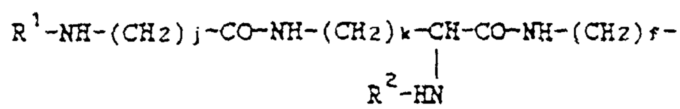

und die farbigen oder fluoreszierenden Markierungen an den Träger im Zuge der Peptidsynthese angeknüpft werden;

(i) die Probe wird den Operationen (i) und (ii) von Anspruch 10 unterworfen;
(ii) die Proben wird der Operation (xiii) von Anspruch 10 unterworfen;
(iii) Proben A (sofern welche übrigbleiben) werden nacheinander den Operationen (iii), (i), (ii) von Anspruch 10 unterworfen, anschließend werden die resultierenden A-Proben in dem nächsten Operationszyklus verwendet;
(iv) Proben B werden der Operation (x) von Anspruch 10 unterworfen, dann gemischt und den Operationen (i) und (ii) von Anspruch 10 unterworfen, anschließend werden die resultierenden Proben n-r aufeinanderfolgenden Operationszyklen (v) von Anspruch 10 unterworfen, anschließend werden die Proben nacheineinander analysiert;
(v) Operationszyklus (ii) bis (iv) dieses Anspruchs wird n-2 mal wiederholt;
(vi) alle Proben werden einem Entschützen unterworfen.

**24.** Verfahren zur Bestimmung der Aminosäuresequenzen von bioaktiven Peptiden ("Dominostrategie") unter Verwendung von Testsätzen gemäß Ansprüchen 1 bis 9, wobei die Verwendung von unmarkierten Subtestsätzen drei aufeinanderfolgende Schritte umfaßt;

(i) Bestimmung der Aminosäure-Ereignis- bzw. Erscheinungsliste durch Ausführen von Screening-Experimenten mit allen Komponenten des Aminosäuretestsubstanz-Subtestsatzes;
(ii) Bestimmung der Ereignisbibliothek durch Ausführen von Screening-Experimenten mit ausgewählten Komponenten des ersten Sub-Testsatzes;
(iii) Bestimmung der Aminosäuresequenzen durch die Domino-ähnliche Verwendung im Screening von ausgewählten Komponenten des (der) zweiten Sub-Testsatzes (-sätze) der Stammpeptidbibliothek oder durch Synthese des zweiten Sub-Testsatzes der Ereignisbibliothek und Ausführen von Screening-Tests mit seinen Komponenten;

während unter Verwendung markierter Testsätze die in (i) beschriebenen Experimente weggelassen werden können, die von (ii) mit allen Komponenten des Sub-Testsatzes ausgeführt werden und (iii) unverändert angewendet wird.

**Revendications**

**1.** Trousse de mélanges de peptides pour la détermination des séquences d'aminoacides de peptides bioactifs dans une banque de peptides apparentés, qui comprend

une "première trousse secondaire" qui est constituée d'un jeu complet de banques secondaires de première ordre de ladite banque de peptides apparentés et
une "seconde trousse secondaire" de mélanges de peptides, se composant d'un jeu complet de banques secondaires de type vicinal et disjoint du second ordre, éventuellement d'ordre supérieur de la même banque de peptides apparentés, ou d'une banque partielle (banque d'occurrence) de la même banque de peptides apparentés et comprenant éventuellement aussi une trousse secondaire d'essai d'aminoacides ("troisième trousse secondaire"), constituée de composants combinés de la première trousse secondaire.

**2.** Trousse suivant la revendication 1, caractérisée en ce que les composants de la première trousse secondaire sont des banques partielles spéciales de la banque de peptides apparentés où le même aminoacide occupe une certaine position dans tous les peptides, cependant que dans le reste des positions, les aminoacides varient comme dans la banque de peptides apparentés.

**3.** Trousse suivant la revendication 1, caractérisée en ce que les composants de la seconde trousse secondaire sont des banques partielles spéciales de la banque de peptides apparentés où les mêmes deux positions sont occupées par les mêmes deux aminoacides dans tous les peptides, cependant que dans le reste des positions, les aminoacides varient comme dans la banque de peptides apparentés.

**4.** Trousse suivant la revendication 1, caractérisée en ce que les composants de la trousse secondaire d'essai d'aminoacides est formée de mélanges combinés de tous ceux des composants de la première trousse secondaire où la position non variée est occupée par le même aminoacide.

**5.** Trousse suivant l'une quelconque des revendications 1 à 4, dans laquelle le nombre de restes d'aminoacides des peptides des banques de peptides apparentés varie dans la plage de 2 à 25.

**6.** Trousse suivant l'une quelconque des revendications 1 à 5, dans laquelle les sous-unités d'aminoacides de peptides sont des L-aminoacides, des D-aminoacides, des aminoacides inhabituels et/ou des dérivés d'aminoacides.

**7.** Trousse suivant l'une quelconque des revendications 1 à 6, caractérisée en ce qu'elle comprend des mélanges de peptides libres, ou de peptides liés à un support solide marqué.

8. Trousse de peptides libres suivant l'une quelconque des revendications 1 à 7, dans laquelle les groupes marqueurs colorés et/ou fluorescents sont attachés aux terminaisons C et/ou N de peptides.

9. Trousse suivant l'une quelconque des revendications 1 à 7, dans laquelle les composants des trousses sont des mélanges de banques secondaires attachées à des supports solides qui diffèrent par la couleur et/ou la fluorescence et/ou la densité et/ou le calibre des perles, où la couleur et/ou la fluorescence et/ou la densité et/ou le calibre des perles se rapportent à (i) la position de couplage de l'aminoacide non varié, (ii) l'aminoacide occupant la position non variée, (iii) à la fois l'aminoacide non varié et sa position, rendant ainsi les banques secondaires mélangées discernables.

10. Procédé de synthèse de trousses de banques secondaires de peptides suivant l'une quelconque des revendications 1 à 9, caractérisé en ce qu'il comprend la combinaison et/ou la répétition d'au moins l'une des opérations qui suivent :

(i) la subdivision en *p* portions égales d'un support solide, ou d'un support aminoacylé, ou d'un support aminoacylé mixte, ou d'un support portant des peptides, ou d'un support portant un mélange de peptides où *p* est le nombre d'aminoacides variés dans la banque de peptides apparentés dans une position de couplage donnée,
(ii) chacune des portions *p* est soumise à l'élimination du groupe protégeant la fonction amino terminale et le couplage à un aminoacide protégé différent,
(iii) le mélange des portions *p,*
(iv) l'enlèvement de la partie 1/ (*n-r*+1) de chacune des portions *p* donnant des échantillons B et les restes qui sont appelés (s'il en subsiste) échantillons A, où *r* et *n* représentent le nombre de restes d'aminoacides déjà attachés au support et le nombre de restes d'aminoacides dans les peptides de la banque de peptides apparentés, respectivement,
(v) exécution des opérations (i) à (iii) sur chacun des échantillons *p,*
(vi) exécution des opérations (i)-(ii) sur chacune des portions *p,*
(vii) deux jeux d'échantillons B, chaque jeu comprenant *p* échantillons, sont combinés en paires contenant le même reste d'aminoacide dans la position non variée des peptides pour former *p* échantillons combinés, appelés échantillons B,
(viii) couplage d'un aminoacide protégé différent à chacun des portions *p,*
(ix) exécution d'opérations (i) à (iii) sur un échantillon,
(x) soumission de chacune des portions *p* à l'enlèvement du groupe protégeant $R^2$ du support et au couplage avec un marqueur coloré ou fluorescent différent,
(xi) enlèvement du groupe protégeant la fonction amino terminale et couplage d'un marqueur coloré ou fluorescent différent à chacune des portions *p*,
(xii) subdivision de chacun de *p* échantillons égaux en *p* portions égales,
(xiii) enlèvement de 1/ (*n-r*) partir de chacune de *p* portions, par ailleurs le même que dans le cas de l'opération (iv),
(xiv) soumission de chacune de *p* portions à l'enlèvement du groupe protecteur $R^2$ du support et au couplage avec un marqueur coloré ou fluorescent différent,
(xv) enlèvement de 1/(*n-r*-1) partie de chacun de *p* portions, par ailleurs le même que dans le cas de l'opération (iv).

11. Procédé de synthèse de la première trousse secondaire suivant la revendication 10, qui comprend des peptides libres non marqués, où le procédé est amorcé avec *p* échantillons égaux de support O-aminoacylé N-protégé, tous les échantillons différant par le reste aminoacyle,

(i) en premier lieu, les échantillons sont soumis à l'opération (iv) de la revendication 10, puis des échantillons A sont consécutivement soumis à des opérations (iii), (i) et (ii) de la revendication 10 et les mélanges résultants sont utilisés comme échantillons de départ, dans le cycle suivant d'opérations,
(ii) des échantillons B sont soumis (*n-r*) fois à l'opération (v) de la revendication 10, puis les échantillons sont pris à part,
(iii) le cycle d'opérations (i) à (ii) de cette revendication est répété (*n-2*) fois,
(iv) tous les échantillons sont soumis à une déprotection et une séparation d'avec le support.

12. Procédé suivant la revendication 10 de préparation d'une première trousse secondaire de peptides libres portant des marques colorées ou fluorescentes à leur terminaison C, attribuées aux restes d'aminoacides occupant les

positions non variées,

(i) le procédé est amorcé avec *p* échantillons égaux de supports solides comprenant des groupes O-aminoacylés N-protégés où le groupe aminoacylé est un marqueur coloré ou fluorescent différant par la couleur ou la fluorescence de la marque du support,
(ii) les échantillons sont soumis à l'opération (xiii) de la revendication 10,
(iii) des échantillons A (s'il en subsiste) sont soumis à l'opération (v) de la revendication 10, puis sont utilisés comme échantillons de départ dans le cycle suivant d'opérations,
(iv) des échantillons B sont soumis à l'opération (ii) de la revendication 10, puis mélangés et soumis consécutivement (*n-r*) fois à l'opération (ix) de la revendication 10, puis l'échantillon est pris à part,
(v) des opérations (ii) à (iv) de cette revendication sont répétées (*n*-1) fois,
(vi) tous les échantillons sont soumis à une déprotection et une séparation d'avec le support.

**13.** Procédé de synthèse d'une première trousse secondaire de peptides libres portant des marques colorées ou fluorescentes à leur terminaison C, attribuées aux restes d'aminoacides occupant les positions non variées, où le procédé est amorcé avec un échantillon de support solide comprenant des radicaux hydroxyméthyle O-acylés correspondant à la formule générale suivante dans laquelle $R^1$ et $R^2$ sont des radicaux protecteurs, *j*, *k* et *f* sont des nombres entiers dont la valeur varie de 1 à 6,

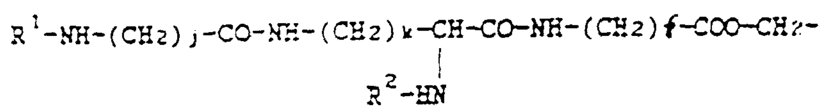

(i) l'échantillon est soumis aux opérations (i) et (ii) de la revendication 10,
(ii) les échantillons sont ensuite soumis à l'opération (iv) de la revendication 10,
(iii) des échantillons B sont soumis à l'opération (x) de la revendication 10, puis les échantillons sont mélangés et soumis (*n-r*) fois à l'opération (ix) de la revendication 10 et l'échantillon est pris à part,
(iv) des échantillons A (s'il en subsiste) sont mélangés et ensuite soumis aux opérations (i) et (ii) de la revendication 10, puis ils sont utilisés comme échantillons de départ pour le cycle suivant d'opérations,
(v) on répète (*n*-1) fois le cycle d'opérations (ii) à (iv) de cette revendication,
(vi) tous les échantillons sont soumis à une déprotection et une séparation d'avec le support.

**14.** Procédé de synthèse d'une première trousse secondaire de peptides libres portant des marques colorées ou fluorescentes à leur terminaison N, attribuées aux restes d'aminoacides occupant les positions non variées, où le procédé est amorcé avec *p* échantillons égaux de support O-aminoacylés N-protégé, tous les échantillons différant par le reste aminoacyle,

(i) en premier lieu, les échantillons sont soumis à l'opération (iv) de la revendication 10,
(ii) des échantillons B sont (*n-r*) fois consécutivement soumis à l'opération (v) de la revendication 10, puis à une opération (xi) de la revendication 10 et, après le mélange, l'échantillon est pris à part,
(iii) des échantillons A (s'il en subsiste) sont consécutivement soumis aux opérations (iii), (i) et (ii) de la revendication 10 et les mélanges résultants sont utilisés comme échantillons de départ dans le cycle suivant d'opérations,
(iv) on répète (*n*-1) fois le cycle d'opérations (i) à (iii) de cette revendication,
(v) tous les échantillons sont soumis à une déprotection et une séparation d'avec le support.

**15.** Procédé de synthèse d'une trousse secondaire d'aminoacides de peptides libres, comprenant les étapes suivantes :

(i) on amorce le procédé avec *p* échantillons égaux de support O-aminoacylé N-protégé, tous les échantillons différant par le reste acylamino,
(ii) en premier lieu, les échantillons sont soumis à l'opération (iv) de la revendication 10,
(iii) sur des échantillons A (s'il en subsiste), on réalise consécutivement les opérations (iii), (i) et (ii) de la revendication 10, puis on utilise les échantillons dans le cycle suivant d'opérations,
(iv) des échantillons B, en même temps que les échantillons B résultants du cycle précédent (s'il a été réalisé), sont soumis à l'opération (vii) de la revendication 10,

(v) si $r < n$ échantillons B sont soumis à une opération (v) de la revendication 10, ils sont par ailleurs pris à part,
(vi) on répète ($n$-1) fois le cycle comprenant les opérations (ii) à (v) de cette revendication,
(vii) finalement, les échantillons B résultants sont soumis à déprotection et à séparation d'avec le support.

**16.** Procédé de synthèse d'une trousse secondaire d'essai d'aminoacides de peptides liés à un support non marqué, comprenant les étapes suivantes :

(i) le procédé est amorcé avec un échantillon de support solide, portant des radicaux amino primaires bloqués attachés par un espaceur,
(ii) l'échantillon est en premier lieu consécutivement soumis à déprotection et aux opérations (i) et (viii) de la revendication 10,
(iii) ensuite, les échantillons sont traités selon les opérations (ii) à (vi) de la revendication 16,
(vi) finalement, les échantillons B résultants sont soumis à déprotection.

**17.** Procédé de synthèse d'une première trousse secondaire d'une banque de peptides apparentés normaux liée à un support coloré et/ou fluorescent, où la couleur, ou la fluorescence, ou le calibre, ou la densité des perles, ou des combinaisons de ces caractéristiques sont attribuées aux restes d'aminoacides occupant la position non variée,

(i) le procédé est amorcé avec $p$ échantillons de supports en polymère et en verre, de calibres de perles différents, comprenant des radicaux amino primaires libres ou protégés, où les perles sont colorées ou fluorescentes, différant par au moins l'une des caractéristiques mentionnées,
(ii) les échantillons sont soumis aux opérations (ii) à (v) de la revendication 13,
(iii) finalement, tous les échantillons sont soumis à déprotection.

**18.** Procédé de synthèse d'une première trousse secondaire liée à des supports colorés et/ou fluorescents, en utilisant un support solide comprenant des groupes acylaminoalkyle correspondant à la formule générale suivante dans laquelle $R^1$ et $R^2$ sont des radicaux protecteurs, $j$, $k$ et $f$ sont des nombres entiers dont la valeur varie de 1 à 6,

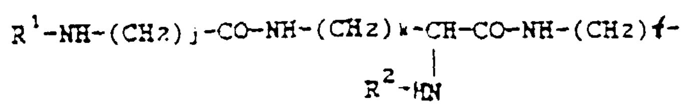

et les marqueurs colorés ou fluorescents attribués aux restes d'aminoacides occupant la position non variée sont attachés au support, au cours de la synthèse des peptides,

(i) on amorce la synthèse avec un échantillon de support,
(ii) on soumet l'échantillon aux opérations (i) à (v) de la revendication 14,
(iii) finalement, tous les échantillons sont soumis à déprotection.

**19.** Procédé de synthèse d'une trousse secondaire, comprenant toutes les banques secondaires de type vicinal du second ordre d'une banque de peptides apparentés de peptides solubles non marqués, où l'on amorce le procédé avec $p$ échantillons égaux de support du type O-acylamino, tous les échantillons différant par le reste acylamino,

(i) on soumet les échantillons à l'opération (xiii) de la revendication 10,
(ii) les échantillons A (s'il en subsiste) sont consécutivement soumis aux opérations (iii), (i), (ii) de la revendication 10, puis les échantillons A résultants sont utilisés dans le cycle suivant d'opérations,
(iii) des échantillons B sont soumis à l'opération (vi) de la revendication 10, puis les échantillons résultants non mélangés sont un par un soumis à $n$-$r$ cycles consécutifs d'opérations (i) à (iii) de la revendication 10,
(iv) tous les échantillons résultants tirant leur origine des échantillons B sont pris à part,
(v) on répète $n$-2 fois le cycle d'opérations (i) à (iv) de cette revendication,
(vi) tous les échantillons sont soumis à séparation et déprotection.

**20.** Procédé de synthèse d'une trousse secondaire liée à un support non marqué, comprenant toutes les banques secondaires du type vicinal de second ordre d'une banque de peptides apparentés, où un échantillon de support solide, portant des radicaux amino primaires bloqués attachés à un espaceur, est consécutivement soumis à déprotection, des opération (i) et (viii) de la revendication 10, puis les échantillons sont traités selon les opérations

(i) à (v) de la revendication 19 et finalement, tous les échantillons sont soumis à déprotection.

**21.** Procédé de synthèse d'une trousse secondaire comprenant toutes les banques secondaires de type vicinal du second ordre d'une banque de peptides apparentés de peptides solubles, portant des marques colorées ou fluorescentes à leur terminaison C, attribuées au reste d'aminoacide occupant la position de couplage inférieure des deux positions vicinales non variées, où on amorce le procédé avec *p* échantillons égaux de support solide, comprenant des radicaux O-aminoacyle N-protégés, où le groupe aminoacyle est un marqueur coloré ou fluorescent, différant par la couleur ou la fluorescence de la marque du support,

(i) en premier lieu, on réalise l'opération (xv) de la revendication 10,
(ii) des échantillons A (s'il en subsiste) sont soumis à l'opération (v) de la revendication 10, puis les échantillons A résultants sont utilisés dans le cycle suivant d'opérations,
(iii) des échantillons B sont consécutivement soumis à des opérations (ii), (iii), (i) et (ii) de la revendication 10, puis les échantillons résultants sont soumis à *n-r* cycles consécutifs d'opération (v) de la revendication 10, puis les échantillons sont pris à part,
(iv) on répète (*n*-2) fois le cycle d'opérations (i) à (iii) de cette revendication,
(v) tous les échantillons sont soumis à séparation et déprotection.

**22.** Procédé de synthèse d'une trousse secondaire comprenant toutes les banques secondaires de type vicinal du second ordre d'une banque de peptides apparentés normaux liée à des supports colorés ou fluorescents, lorsque la couleur ou la fluorescence, ou le calibre, ou la densité du support est attribuée au reste d'aminoacide occupant la position de couplage inférieure de deux positions vicinales non variées et où le procédé est amorcé avec *p* échantillons égaux de supports en polymère et en verre comprenant des radicaux amino primaire libres ou protégés, où les perles de calibre différent sont colorées et fluorescentes, différant par au moins l'une des caractéristiques mentionnées du support, puis les échantillons sont soumis à des opérations (i) à (iv) de la revendication 21, finalement tous les échantillons sont soumis à déprotection.

**23.** Procédé de synthèse d'une trousse secondaire comprenant toutes les banques secondaires de type vicinal du second ordre d'une banque de peptides apparentés normaux liée à des supports colorés ou fluorescents, lorsque la couleur ou la fluorescence du support est attribuée au reste d'aminoacide occupant la position de couplage inférieure des deux positions vicinales non variées et où le procédé est amorcé avec un seul échantillon déprotégé de support solide, comprenant des radicaux acylaminoalkyle correspondant à la formule générale suivante dans laquelle $R^1$ et $R^2$ sont des radicaux protecteurs, *j*, *k* et *f* sont des nombres entiers dont la valeur varie de 1 à 6,

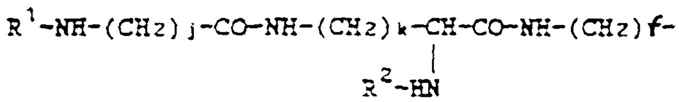

et les marques colorées ou fluorescentes sont attachées au support, au cours de la synthèse des peptides,

(i) l'échantillon est soumis aux opérations (i) et (ii) de la revendication 10,
(ii) les échantillons sont soumis à l'opération (xiii) de la revendication 10,
(iii) des échantillons A (s'il en subsiste) sont consécutivement soumis à des opérations (iii), (i), (ii) de la revendication 10, puis les échantillons résultants A sont utilisés dans le cycle suivant d'opérations,
(iv) des échantillons B sont soumis à l'opération (x) de la revendication 10, puis sont mélangés et soumis aux opérations (i) et (ii) de la revendication 10, puis les échantillons résultants sont soumis à *n-r* cycles consécutifs d'opération (v) de la revendication 10, puis les échantillons sont pris à part,
(v) on répète *n*-2 fois le cycle d'opérations (ii) à (iv) de cette revendication,
(vi) tous les échantillons sont soumis à déprotection.

**24.** Méthode de détermination des séquences d'aminoacides de peptides bioactifs ("stratégie de domino"), en utilisant des trousses suivant l'une quelconque des revendications 1 à 9, conformément à laquelle
l'utilisation de trousses secondaires non marquées comprend trois étapes consécutives :

(i) détermination de la liste d'occurrence des aminoacides par l'exécution d'expériences de criblage ou de triage avec tous les composants de la trousse secondaire d'essai d'aminoacides,

(ii) détermination de la banque d'occurrence en entreprenant des expériences de criblage ou triage avec des composants choisis de la première trousse secondaire,
(iii) détermination des séquences d'aminoacides par le domino, comme l'utilisation pour le criblage ou triage de composants choisis de la ou des secondes trousses secondaires de la banque de peptides apparentés, ou par la synthèse de la seconde trousse secondaire de la banque d'occurrence et l'entreprise d'essais de triage ou criblage avec ses composants,

cependant que lorsque l'on utilise des trousses marquées, les expériences décrites en (i) peuvent être omises, celles de (ii) sont entreprises avec tous les composants de la première trousse secondaire et (iii) s'applique de manière inchangée.